(19) Europäisches Patentamt
European Patent Office
Office européen des brevets

(11) EP 3 677 244 A2

(12) EUROPEAN PATENT APPLICATION

(43) Date of publication:
**08.07.2020 Bulletin 2020/28**

(21) Application number: **20155494.6**

(22) Date of filing: **04.02.2020**

(51) Int Cl.:
***A61K 8/14*** (2006.01) ***A61Q 19/10*** (2006.01)
***A61Q 5/02*** (2006.01) ***A61Q 5/12*** (2006.01)
***A61K 8/36*** (2006.01) ***A61K 8/37*** (2006.01)
***A61K 8/39*** (2006.01) ***A61Q 13/00*** (2006.01)
***C11D 3/50*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Clariant International Ltd**
**4132 Muttenz (CH)**

(72) Inventors:
- **BREFFA, Catherine**
**68161 Mannheim (DE)**
- **COHRS, Carsten**
**60316 Frankfurt am Main (DE)**

(74) Representative: **Kampen, Daniela**
**Clariant Produkte (Deutschland) GmbH**
**Patent & License Management Chemicals**
**Industriepark Höchst / G 860**
**65926 Frankfurt am Main (DE)**

# (54) COMPOSITIONS COMPRISING MULTILAMELLAR VESICLES

(57) The invention relates to compositions, in particular cosmetic compositions or fabric conditioner compositions, comprising multilamellar vesicles comprising fragrance. The invention also relates to the use of multilamellar vesicles comprising fragrance in cosmetic compositions or fabric conditioner compositions.

**Figure 1**

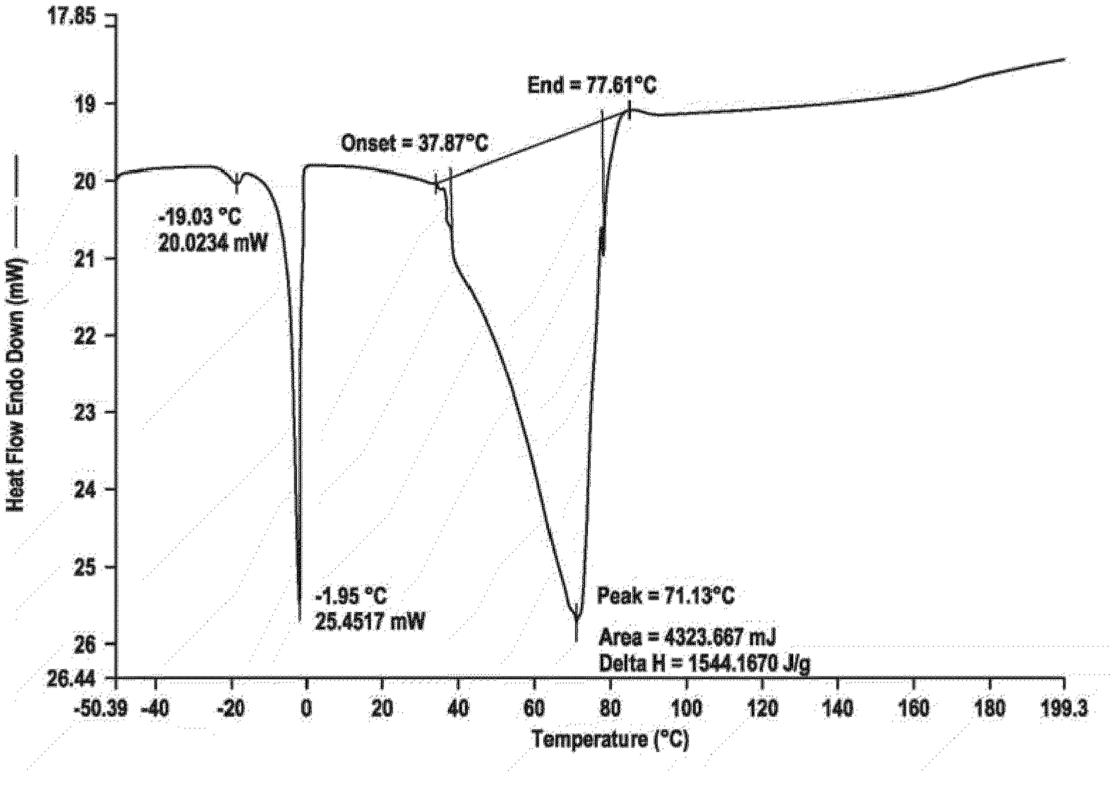


EP 3 677 244 A2

## Description

The invention relates to compositions, in particular cosmetic compositions or fabric conditioner compositions, comprising multilamellar vesicles comprising fragrance. The invention also relates to the use of multilamellar vesicles comprising fragrance in cosmetic compositions or fabric conditioner compositions.

Many cosmetic and fabric softener compositions comprise fragrances. These are usually mixed directly into the compositions, for example shampoos, hair conditioners, shower gels, face cleansers, solid or liquid soaps or creams and lotions and leave on products in haircare. This procedure has the disadvantage that in most cases upon use only small amounts of the fragrance remain on the skin, on the hair or on the fabric, which can develop their effect there. The majority of the fragrances is usually washed off during use. This leads to large amounts of costly fragrances having to be incorporated into the formulations in order to achieve a desired effect. However, when suitably high amounts of fragrances are used in cosmetic or fabric softener formulations, this may lead to an undesired skin irritation when the formulations are used.

Fragrances are volatile substances. Various approaches of encapsulation have already been used to avoid premature delivery of fragrances. Examples thereof are polymeric encapsulation or inorganic encapsulation. These approaches have been carried out for the development of long-lasting fragrance delivery systems. Polymeric capsules such as melamine formaldehyde, polyacrylates or polyurethanes usually result in microcapsules with particle size above 1 micron with pressure triggered release in various applications. There still exist challenges to encapsulate a broad range of fragrances. The pressure triggered capsules rather release the fragrances quickly with friction, hence, fragrance long-lasting is not prolonged. Diffusion controlled release would be desired for the prolonged fragrance long-lasting in cosmetic and fabric softener products.

There is still demand for cosmetic or fabric conditioner formulations which, on the one hand, allow the amount of fragrances used to be kept low and thus to reduce costs, but which, on the other hand, nevertheless allow a very good effectiveness of the fragrances and are storage stable.

If the effectiveness of the fragrances could be increased during use, it would be possible to use smaller amounts, meaning that the cosmetic or fabric conditioner formulations could be produced more cost-effectively.

WO 2019/115621 A1 discloses that fragrance molecules over a broad range of partition coefficients ("Log P") can be encapsulated in multilamellar vesicles comprising two or more concentric lipid double layers. These vesicles are submicron particles with narrow particle size distribution and with high encapsulation efficiency of fragrance. The encapsulated fragrance is stable under storage conditions and will have long-lasting fragrance release on use thereof.

The present invention relates to a composition, in particular cosmetic composition or fabric conditioner composition, comprising multilamellar vesicles and one or more further components (F),
wherein the multilamellar vesicles are in the shape of a rotational body and comprise one or more (preferably two or more) concentric lipid double layers and fragrance,
wherein the multilamellar vesicles have a mean diameter between 100 and 800 nm, and
wherein the lipid double layers comprise

a) at least one surfactant having a HLB value of greater than 6, and
b) an amphiphilic compound having a logP value of 1 or above, and

wherein the multilamellar vesicles comprise in addition to components a) and b) a fragrance having a logP value of 1 or above.

Surfactant of component a) is characterized by its HLB value of more than 6. The nature of a surfactant is represented by the hydrophilic-lipophilic balance of the molecule. The degree of this hydrophilic-lipophilic balance can be determined by calculating values for the different regions of the molecule, as described by Griffin in 1949 and 1954. Griffin's method has been primarily developed for non-ionic surfactants as described in 1954 works as follows

$$HLB = 20 * M_h/M$$

where $M_h$ is the molecular mass of the hydrophilic portion of the molecule, and M is the molecular mass of the whole molecule, giving a result on a scale of 0 to 20. An HLB value of 0 corresponds to a completely lipophilic molecule, and a value of 20 corresponds to a completely hydrophilic molecule.

The term "HLB" as used in this specification for nonionic surfactants is calculated by the above formula. The method of Griffin is published, for example, in Journal of the Society of Cosmetic Chemists, 5 (4), 249-256 (1954).

The term "HLB" as used in this specification for anionic, cationic or amphoteric surfactants is calculated by the method of Davies. This method is published, for example, in Gas/Liquid and Liquid/Liquid Interfaces. Proceedings of

2nd International Congress Surface Activity, pp. 426-438, Butterworths, London 1957

Amphiphilic compound of component b) is characterized by its log P value of ≥ 1. The amphiphilic character of component b) can be determined by its partition coefficient between octanol and water. The octanol-water partition coefficient (log P) is a measure of the distribution of a substance between the aqueous and the organic octanol phase and is defined as follows

$$\log\ P_{\text{oct/wat}} = \log\left(\frac{[\text{solute}]_{\text{octanol}}^{\text{un-ionized}}}{[\text{solute}]_{\text{water}}^{\text{un-ionized}}}\right)$$

Examples of calculated and measured log P values are found in A. Leo, C. Hansch, D. Elkins, Chemical Reviews, Volume 71, no. 6, (1971).

The vesicles used in the present invention have a shape of a rotational body, such as that of a sphere or an ellipsoid or that of a disk or other shape of a solid of revolution. Preferably, the shape of the vesicles is spherical, ellipsoidal or disk-like.

The mean diameter of the vesicles used in the present invention is between 80 and 800 nm, preferably between 100 and 500 nm and most preferably between 150 and 400 nm. Also preferably, the mean diameter of the vesicles used in the present invention is between 100 and 200 nm. The mean diameter is determined by laser diffraction analysis, for example by using a Horiba LA 940 or Mastersizer 3000 from Malvern using the "Mie Scattering Theory" evaluation. In case of vesicles having axes of different length, such as vesicles having the shape of an ellipsoid or of a disk, the largest axis determines the mean diameter.

The vesicles used in the present invention have a narrow particle size distribution of Gaussian shape. Preferably, the standard deviation of the particle size distribution is between 10 % and 90 % of the mean diameter.

The vesicles used in the present invention contain at least one fragrance having a log P value of 1 or above. The term "log P" has been defined above. The fragrance is an additional component present in the vesicles besides components a) and b).

The vesicles used in the present invention may incorporate high amounts of one or more fragrances, for example more than 30 % by weight, referring to the total amount of the vesicle. But vesicles having lower amounts of fragrance(s) are also possible.

The vesicles used in the present invention may optionally contain co-surfactants as component c) in addition to components a), b) and fragrances. Co-surfactants are surfactants which are not capable of forming micelles. A co-surfactant is any amphiphilic substance having a HLB value ≤ 6. Preferred co-surfactants have a HLB-value between from 2 to 6.

The vesicles used in the present invention may optionally contain waxes as component d) in addition to components a), b) and fragrances or in addition to components a), b), c) and fragrances. Preferred are vesicles containing fragrances and components a), b) and c). Preferred are vesicles containing fragrances and components a), b) and d). Preferred are vesicles containing fragrances and components a), b), c) and d).

Due to the buffer systems of the non-fragrance amphiphilic compounds present in the vesicles used in the invention, the retarded release of different fragrances or fragrance components has similar characteristics.

The multilayer(s) of the vesicles used in the present invention can adopt a solid gel phase state at lower temperatures but may undergo phase transition to a fluid state at higher temperatures, and the chemical properties of the amphiphilic compounds constituting such multilayers influence at which temperature this will happen. For controlling the retarded release of the fragrance, a solid gel state of the multilayer(s) is preferred. The temperature for the phase transition depends very much on the solidification point of the amphiphilic components in the lipid multilayer(s). The temperature for the phase transition can be determined, for example, by differential scanning calorimetry (DSC).

For the use in fabric softeners a high transition temperature is required, because laundry is often dried at a high heat in a tumble-drier.

By proper choice of the above-mentioned amphiphilic substances a), b), c) and/or d) or other non-fragrance amphiphilics, the temperature for the phase transition from a solid gel phase state to a fluid state may be modified within a broad temperature range.

Preferred are vesicles having a phase transition from a solid gel phase state to a fluid state within a range from 30 °C to 90 °C, preferably from 35 °C to 80 °C. This temperature range is preferably chosen for optimum release kinetics and protection of the vesicle against heat.

The lipid double layers of the vesicles used in the present invention are formed from a selected combination of fragrances with surfactants and amphiphilic compounds, defined above as components a) and b) which may optionally contain in addition components c) and/or d).

Mixtures of fragrances with selected components a) and b) and optionally with components c) and/or d) are used which form multilamellar liquid-crystalline structures. Such structures can be determined by means of optical microscopy using a polarization microscope. In addition, multilamellar liquid-crystalline structures can be determined by TEM or TEM-freeze fracture technology. Appropriate techniques are known to the person skilled in the art.

The mixtures of fragrances with components a) and b) and optionally with components c) and/or d) forming the vesicles used in this invention are chosen so that a multilamellar liquid-crystalline structure is formed. The selection of suitable amounts of the fragrances and components a) and b) and optionally with components c) and/or d) is possible through simple manual experiments.

The mixtures of fragrances with components a) and b) and optionally with components c) and/or d) forming the vesicles used in the invention are chosen so that in water or selected aqueous media multilamellar vesicles are obtainable which have an average diameter of less than 800 nm. The aqueous media may contain additional additives, such as electrolytes, polyols such as glycerin, polyethylene glycol or propylene glycol, or water-soluble vitamins.

The vesicles used in the invention contain fragrances and components a) and b) and optionally components c) and/or d) which are able to form lyotropic lamellar liquid-crystalline phases. The formation of liquid-crystalline structures is essentially dependent on the geometry of fragrances, components a) and b) and optional components c) and/or d), which can be expressed by the packing parameter PP.

$$PP=V_0 / (a_e * l_0)$$

$V_0$ surfactant tail volume
$a_e$ equilibrium area per molecule at the aggregate interface
$l_0$ tail length

A packing parameter PP can be assigned to a chemical species, for example to a surfactant of component a), b), c) or d) or to a fragrance.

If several chemical species are present in certain concentrations to form a mixture of these species, a packing parameter of this mixture PPmixture can be calculated.

A packing parameter of a mixture is defined by the following formula:

$$PP_{mixture} = (\sum c_i * PP_i) / c_{total,}$$

wherein

| | |
|---|---|
| $PP_i$ | is the packing parameter of the single species i, |
| $c_i$ | is the concentration of the single species i in weight percent, and |
| $C_{total}$ | is the total concentration of all i species in the mixture. |

Depending on their packing parameter, components a) and b) are forming different aggregates. Lyotropic spherical lamellar liquid-crystalline structures as required for entrapping fragrances are formed by components a) and b) optionally in combination with components c) and/or d) at a resulting packing parameter PPmixture of at least 0.5 and preferably in the range between 0.5 and 1.

In a preferred embodiment, the vesicles comprise components a), b) and optionally component c) and/or component d) and the packing parameter of the mixture of components a), b) and optionally component c) and/or component d) has a value of 0.5 or above, more preferred in the range of 0.5 to 1.

Compounds with packing parameters PP or PPmixture <0.5 form micelles. However, micelles are present in a dynamic equilibrium and continually breakdown and build up again. For this reason, micelles are not very suitable as storage media for other ingredients. As the packing parameter is shifting into a range of 0.3 - 0.5, the compounds or mixtures of compounds form rod-like micelles. Compounds or compound blends with packing parameters > 0.5 - < 1 form preferably vesicles. Sandwich double layers are preferably formed at packing parameters around 1. According to the invention, then, components a) and b) and optionally components c) and/or d) are used which may be present in spherical lyotropic lamellar liquid-crystalline phases. In the lyotropic state, fragrance molecules are stored, for example, between the components forming the required vesicle structure. The hydrophilic moiety of a component can be varied according to the desired adhesion to a later substrate. For example, the hydrophilic moiety can be varied for adhesion to the human skin or to textile fibers.

Vesicles used in the present invention are preferably formed when the packing parameter of the mixture of all

participating surfactants and amphiphilic molecules is 0.5 or above, more preferred in the range 0.5 - 1. This range is valid for spherical vesicles. If the shape of the vesicles is ellipsoid or disk like, the packing parameter value shifts to higher values.

HLB values may be correlated to the packing parameter and to Log P values. Therefore, it is assumed that the fragrance molecules encapsulated in the vesicles are a part of the bilayers.

The incorporation of fragrances into the multilamellar liquid-crystalline structure of the vesicles will modify the value of the packing parameter of the mixture of the vesicle loaded with the fragrance.

The packing of lipids within the bilayer also affects its mechanical properties, including its resistance to stretching and bending and including the release kinetics and/or release concentration of the encapsulated fragrances.

The vesicles used in the present invention can encapsulate fragrances with a very broad log P range, provided components a) and b) and optionally c) and/or d) are present in the multilamellar liquid-crystalline structure.

For matching the required packing parameter of more than about 0.5, preferably of 0.5 to 1.5 and most preferably of 0.5 to 1 for the formation of vesicles with entrapped fragrances, components a) and b) and optionally c) and/or d) are required having a sufficient high packing parameter. The used surfactants of component a) can be of nonionic, anionic, cationic or amphoteric structure. Hence, it is possible to adapt the surface charge of the vesicles to the surface charge of the application area of the fragrance. This allows a maximum deposition of fragrances.

Surfactants of component a) may be nonionic, anionic, cationic or amphoteric surfactants, provided these have a HLB-value of more than 6.

Examples of suitable nonionic surfactants of component a) are polyoxyethylene sorbitan esters, polyoxyethylene sorbitol esters, polyoxyalkylene fatty alcohol ethers, polyoxyalkylene fatty acid esters, alkoxylated glycerides, polyoxyethylene methyl glucoside ester, alkyl polyglucosides, EO-PO blockpolymers or combinations of two or more thereof.

Examples of anionic surfactants of component a) are sulfonates of alkylbenzenesulfonates, alkanesulfonates, olefinsulfonates, alkyl ether sulfate, alkyl sulfate, sulfo-succinates, alkyl phosphates, alkyl ether phosphates, protein fatty acid condensates, perferably collagen hydrolysates modified with fatty acid, amino acid-based surfactants, isethionates, taurides, acyl lactylates, neutralized fatty acids or combinations of two or more thereof.

Examples of cationic surfactants of component a) are esterquats, ditallow dimethyl ammonium chloride, C12/14 alkyl dimethyl benzyl ammonium chloride, alkyl dimethyl benzyl ammonium chloride, cetyl trimethyl ammonium chloride, stearyl trimethyl ammonium chloride, behenyl trimethyl ammonium chloride, alkyl hydroxyethyl dimethyl ammonium chloride, distearyl dimethyl ammonium chloride, dihydrogenated tallow fatty alkyl dimethyl ammonium chloride or combinations of two or more thereof.

Examples of amphoteric surfactants of component a) are alkyl amphoacetate, alkyl amidopropyl betaine, alkyl amidopropyl dimethylamine betaine, undecylenamidopropyl betaine, alkyl dimethyl amine oxide.

Preferred vesicles used in the present invention contain as component a) nonionic surfactants having a HLB-value of more than 6, more preferred polyoxyalkylene fatty alcohol ethers, polyoxyalkylene fatty acid esters, collagen hydrolysates modified with fatty acid or combinations of two or more thereof, most preferred components a) are polyoxyalkylene $C_8$-$C_{24}$-fatty alcohol ethers, polyoxyalkylene $C_8$-$C_{24}$-fatty acid esters, collagen hydrolysates modified with $C_8$-$C_{24}$-fatty acid or combinations of two or more thereof.

Examples of amphiphilic compounds b) are esters of fatty acids, preferably triglycerides of fatty acids or esters of fatty acids and fatty alcohols or combinations thereof.

Triglycerides of fatty acids or esters of fatty acids and fatty alcohols are amphiphilic components which are not capable of forming micelles. Their amphiphilic character is expressed by the Log P value of 1 or above. For a caprylic/capric acid triglyceride, for example, the Log P is 4.

Examples of triglycerides of fatty acids are glycerol esters of one or more fatty acids having between 8 and 24 carbon atoms. Examples of esters of fatty acids with fatty alcohols are esters of fatty acids having between 8 and 24 carbon atoms with fatty alcohols having between 8 and 28 carbon atoms. The fatty acid portions of these esters can be derived from saturated and/or from ethylenically unsaturated aliphatic fatty acids. Unsaturated fatty acids may have one or more ethylenically unsaturated carbon-carbon bonds. Preferably, the triglycerides comprise fatty acid groups from different fatty acids.

Preferred vesicles used in the present invention contain triglycerides of one or more fatty acids having 8 to 24 carbon atoms, preferably 10 to 18 carbon atoms, and/or esters of fatty acids having 8 to 24 carbon atoms with fatty alcohols having 8 to 24 carbon atoms, preferably 10 to 18 carbon atoms, as component b).

Examples of suitable co-surfactants of component c) are sorbitan esters, citric esters, lactic esters, partial fatty acid glycerides, polyglycerides, glycerol esters, polyglycerol esters, sorbitol esters, fatty alcohols, propylene glycol esters, methyl glucoside ester, alkyl polyglucosides, sugar esters or combinations of two or more thereof.

Examples of suitable waxes of component d) are waxes of the monoester type. As waxes are amphiphilic, their amphiphilic behavior may be specified by Log P values. Preferred waxes have Log P values of 4.7 or above, most preferred of 6 or above. As hydrocarbon number increases above C13, as is the case for the majority of the wax constituents, Log P values of 6 or above are found. Waxes are furthermore characterized by their solidification point,

which is typically between 30 and 100 °C. Waxes are organic compounds that characteristically consist of long alkyl chains. They may also include various functional groups such as fatty acids, primary and secondary long chain alcohols, unsaturated bonds, aromatics, amides, ketones, and aldehydes. They frequently contain fatty acid esters as well.

Waxes used as component d) in the present invention can be synthetic ones, animal or plant derived or montan waxes

Optionally, the surfactant mixtures forming the lipid double layers of the vesicles may contain additional polymeric amphiphilic components e) besides the components a), b) and optionally c) and/or d).

Examples of such additional components e) are polymers, such as polyvinyl acetate, polyvinyl pyrrolidone, polyvinyl alcohols, vinyl pyrrolidone (VP)/hexadecene copolymer, VP/ eicosene copolymer or silicone oils and their derivatives. Also these amphiphilic substances can be attributed with a Log P value.

The amount of surfactant(s) of component a) in the lipid double layers of the vesicles used in this invention can vary over a broad range. Typical amounts of these surfactant(s) in the lipid double layers may be between 0.1 and 95 % by weight, referring to the total weight of the lipid double layer, preferably between 10 and 40 % by weight.

The amount of amphiphilic components b) in the lipid double layers of the vesicles used in this invention can vary over a broad range. Typical amounts of components b) in the lipid double layers may be between 0.1 and 95 % by weight, referring to the total weight of the lipid double layer, preferably between 10 and 40 % by weight.

The amount of co-surfactants, component c), in the lipid double layers of the vesicles used in this invention also can vary over a broad range. Typical amounts of co-surfactants in the lipid double layers may be between 0.1 and 50 % by weight, referring to the total weight of the lipid double layer, preferably between 1 and 10 % by weight.

The amount of waxes, component d), in the lipid double layers of the vesicles used in this invention also can vary over a broad range. Typical amounts of these waxes in the lipid double layers may be between 0.1 and 50 % by weight, referring to the total weight of the lipid double layer, preferably between 3 and 10 % by weight.

The amount of additional polymeric amphiphilic components e) in the lipid double layers of the vesicles used in this invention also can vary over a broad range. Typical amounts of components e) may be between 0.1 and 50 % by weight, referring to the total weight of the lipid double layer, preferably between 3 and 30 % by weight.

In the vesicles used in the present invention fragrance molecules with a broad range of LogP, for example in the range of 0.1 - 10, preferably in the range of 1 - 6, can be encapsulated with high encapsulation efficiency. Preferably, the fragrance has a log P value in the range between 1 and 10.

According to the invention, fragrances are understood as meaning fragrant oils. Basic substances of fragrances are generally essential oils, flower oils, extracts from plant and animal drugs, odorants isolated from natural products, chemically modified (semisynthetic) odorants, and odorants obtained by purely synthetic means.

The fragrances can here originate from a large number of plant starting materials. Examples which may be specified are: flowers, for example from lavender, rose, jasmine, neroli; stems and leaves, for example from geranium, patchouli, petit grain, fruits such as anis, coriander, caroway, juniper; fruit peels, for example from agrumes, such as bergamot, lemon, orange; seeds, such as mace, angelica, celery, cardamom; roots, such as angelica,. costus, iris, calmus; wood, such as sandalwood, guaiac wood, cedar wood, rosewood; herbs and grasses, such as tarragon, lemongrass, sage, thyme; needles and branches, for example from spruce, fir, pine, dwarf-pine; resins and balsams, for example from galvanum, elemi, benzoin, myrrh, olibanum, opoponax.

Animal raw materials are, for example, ambergris, musk, civet, castoreum.

Examples of semisynthetic odorants are isoeugenol, vanillin, hydroxycitronellal, citronellol, geranyl acetate, ionones and methylionones. The completely synthetic odorants or fragrances are very diverse and often orientate themselves to natural substances. For a description of the fragrances, reference may be made, for example, to Römpp, Chemielexikon, 9th edition, keywords "parfums [perfumes]", "riechstoffe [odorants]", "duftstoffe [fragrances]". Further suitable fragrances are known to the person skilled in the art.

The fragrances, for example, can be introduced into the spaces between the hydrophobic moieties of the amphiphilic components a), b) and optionally c), d) and/or e), and be stored there. As a result, the fragrances are dissolved, and crystallizing out of the fragrances is prevented. This permits, inter alia, the preparation of cosmetic or fabric softener formulations with a skin-friendly pH, and by preventing the fragrances from crystallizing out, the skin friendliness of the composition is increased further. The mixtures of amphiphilic components used according to the invention having the fragrances dissolved therein spread upon application to the skin, meaning that application of the fragrance to the skin is improved.

Preferably, the vesicles used in the present invention are provided in an aqueous composition in an amount of vesicles of from 0.1 to 60 % by weight of the total amount of the composition, preferably between from 1 to 50 % by weight, most preferably between from 5 to 20 % by weight. The aqueous composition may consist of only water, water and electrolytes or water and polyols or water and alcohols. The polyols may consist of propylene glycol, polyethylene glycol, glycerin, polyglycerin, sorbitol, isosorbide or dimethyl isosorbide.

The vesicles used in the present invention may be prepared by feeding the components constituting the vesicles to an emulsification device for manufacture of nano-emulsions. An example of such emulsification device is disclosed

in US 2013/0201785 A1.

In this document an emulsifying device for continuous production of emulsions and/or dispersions is disclosed which comprises

a) at least one mixing apparatus comprising a rotationally symmetric chamber sealed airtight on all sides, at least one inlet line for introduction of free-flowing components, at least one outlet line for discharge of the mixed free-flowing components, a stirrer unit which ensures laminar flow and comprises stirrer elements secured on a stirrer shaft, the axis of rotation of which runs along the axis of symmetry of the chamber and the stirrer shaft of which is guided on at least one side, wherein the at least one inlet line is arranged upstream of or below the at least one outlet line, wherein the ratio between the distance between inlet and outlet lines and the diameter of the chamber is ≥ 2:1, wherein the ratio between the distance between inlet and outlet lines and the length of the stirrer arms of the stirrer elements is 3:1 to 50:1, and wherein the ratio of the diameter of the stirrer shaft, based on the internal diameter of the chamber, is 0.25 to 0.75 times the internal diameter of the chamber, such that the components introduced into the mixing apparatus via the at least one inlet line are stirred and continuously transported by means of a turbulent mixing area on the inlet side, in which the components are mixed turbulently by the shear forces exerted by the stirrer units, a downstream percolating mixing area in which the components are mixed further and the turbulent flow decreases, a laminar mixing area on the outlet side, in which a lyotropic, liquid-crystalline phase is established in the mixture of the components, in the direction of the outlet line,
b) at least one drive for the stirrer unit and
c) at least one conveying device per component or per component mixture.

The vesicles used in the present invention may be prepared by a method which comprises the steps

i) feeding a composition A comprising at least one surfactant of component a) and water to a first inlet line of an emulsification device,
ii) feeding a composition B comprising at least one amphiphilic compound of component b), fragrance and water to a second inlet line of an emulsification device,
iii) combining compositions A and B in a turbulent mixing zone in the emulsification device,
iv) transporting the mixed compositions within the emulsification device towards an outlet line, whereby laminar flow of the mixed components is established in the zone preceding the outlet line, thereby vesicles are formed, and
v) discharging the vesicles via the outlet line form the emulsification device.

Preferably, the vesicles formed in the emulsification device are diluted with water or an aqueous phase. This can be performed in a separate device by introducing the vesicles into water which optionally contains additional surfactants. The aqueous composition may be consisting of only water, water and electrolytes or water and polyols. The polyols may consist of propyleneglycol, polyethylene glycol, glycerin, polyglycerin, sorbitol, isosorbide or dimethyl isosorbide.

Over and above the storage effect, the vesicles used in the invention permit also extensive protection of the fragrances against oxidative decomposition. If appropriate, further antioxidants can also be added. Even without the addition of antioxidants, the fragrances in the vesicles are significantly better protected against oxidation than in conventional forms.

The invention relates to a composition, in particular cosmetic composition or fabric conditioner composition, comprising the multilamellar vesicles described herein and one or more further components (F).

Preferably, the composition of the invention is a cosmetic composition or a fabric conditioner composition. In a preferred embodiment, the composition of the invention is a cosmetic composition. In another preferred embodiment, the composition of the invention is a fabric conditioner composition.

For example, the composition of the invention may be a cosmetic composition selected from the group consisting of shampoo, hair conditioner, body wash, bubble bath, bath oil, facial cleanser, cleansing mask, cleansing milk, micellar water, make-up remover, cleansing wipes, perfume, soaps, shaving soaps, shaving foams and cleansing foams.

Preferred cosmetic compositions are hair care compositions or skin care compositions. Preferably, the composition of the invention is a hair care composition or a skin care composition. More preferred cosmetic compositions are hair care compositions, such as shampoo compositions or hair conditioner compositions. More preferably, the composition of the invention is a hair care composition, such as a shampoo composition or a hair conditioner composition.

Particularly preferably, the composition of the invention is a shampoo composition. The shampoo composition can be in the form of rinse-off products or 'dry shampoo' products, can be opaque or transparent, and can be formulated in a wide variety of product forms, including creams, gels, emulsions, mousses and sprays. Preferably, the shampoo composition of the present invention is in the form of a rinse-off product. The shampoo composition is, for example, suitable for cleansing human hair or animal hair, preferably human hair.

Also particularly preferably, the composition of the invention is a hair conditioner composition. The hair conditioner composition can be in the form of rinse-off products or leave-on products, can be opaque or transparent, and can be formulated in a wide variety of product forms, including creams, gels, emulsions, mousses and sprays. Preferably, the hair conditioner composition is in the form of a rinse-off product.

Typically, the composition of the invention, in particular cosmetic composition or fabric conditioner composition, comprises from 0.1 to 20 wt.-%, preferably from 0.2 to 15 wt.-%, more preferably from 0.3 to 10 wt.-%, even more preferably from 0.5 to 5 wt.-%, particularly preferably from 1 to 3 wt.-% of the multilamellar vesicles described herein, based on the total weight of the composition. For example, the composition of the invention, in particular cosmetic composition or fabric conditioner composition, comprises 1 wt.-%, 2 wt.-%, 3 wt.-%, 4 wt.-%, 5 wt.-%, 6 wt.-%, 7 wt.-%, 8 wt.-%, 9 wt.-%, or 10 wt.-% of the multilamellar vesicles described herein, based on the total weight of the composition.

The composition of the invention, in particular cosmetic composition or fabric conditioner composition, comprises one or more further components (F), which can be in an amount of at least 0.01 % by weight, preferably at least 0.05% by weight, more preferably at least 0.1% by weight, even more preferably at least 0.5% by weight, such as 0.5 to 20% by weight of the composition. Preferably, the component (F) is selected from the group consisting of acidity regulators, colorants, conditioning agents, emulsifiers, film formers, fragrances, glossers, humectants, lubricants, moisturizers, pigments, preservatives, skin penetration enhancers, stabilizers, surfactants, thickeners, and viscosity modifiers. More preferably, the component (F) is selected from the group consisting of acidity regulators, glossers, lubricants, and surfactants.

Suitable lubricants are, for example, fatty alcohol components having 6 to 18 carbon atoms. The surfactants may, for example, be selected from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) or cetylpyridinium chloride.

As cationic components, a variety of further cationic polymers are suitable, including quaternized cellulose ethers, copolymers of vinylpyrrolidone, acrylic polymers, including homopolymers or copolymers of dimethyldiallylammonium chloride or acrylamide. Also suitable are various types of homo- or copolymers derived from acrylic or methacrylic acid, acrylamide, methylacrylamide, diacetone-acrylamide.

In at least one embodiment, the component (F) is a glosser. Typical glossers are silicones. Suitable as silicones are volatile or nonvolatile nonionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, or mixtures thereof.

The composition of the invention may contain from 0.05 to 5%, preferably 0.5 to 5% by weight of at least one oil component. Typical oils are organic oils, which often are esters. The oil component may comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil. Also suitable as the oil component are sorbitan esters.

The composition of the invention can contain from 0.1 to 10% by weight, preferably from 0.2 to 5% by weight, more preferably from 0.2 to 3% by weight, also more preferably from 0.5 to 5% by weight of at least one rheology modifying agent, in particular a gelling and thickening agent. Examples are cellulosic thickeners, for example, hydroxyethylcellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol®. Another class of (meth)acylic acid polymers are alkali-swellable emulsion (ASE) polymers. ASE polymers include, for example, Aculyn® 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration

thereof is increased. In use, hydrodynamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn® 22 and Aculyn® 28 copolymers from Dow and Aqua SF 1® copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a C8-C30 alkyl or, as therein more particularly described, a hydrocarbyl monoether of polyethylene glycol, (3) a C1-C4 alkyl acrylate or methacrylate, and, optionally, (4) a small amount of a polyethylenically unsaturated monomer.

The composition of the invention can also comprise as component (F) a fatty compound. The fatty compound may be included in the composition at a level of from 0.1 to 20 % by weight, preferably from 1.0 to 10 % by weight. The fatty compound is selected from the group consisting of fatty alcohols (e.g. cetyl alcohol, stearyl alcohol or cetearyl alcohol), fatty acids, fatty alcohol derivatives, fatty acid derivatives, or mixtures thereof.

It is understood that the components disclosed can in some instances fall into more than one classification, e.g., some fatty alcohol derivatives can also be classified as fatty acid derivatives. However, a given classification, is not intended to be a limitation on that particular compound but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, or mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, or mixtures thereof. Examples of fatty alcohol derivatives and fatty acid derivatives include methyl stearyl ether, polyoxyethylene ethers of behenyl alcohol, ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, poly glyceryl stearate, glyceryl monostearate, glyceryl distearate, glyceryl tristearate, or mixtures thereof.

The composition of the invention may comprise an aqueous carrier. The level and species of the aqueous carrier are selected according to the compatibility with other components and other desired characteristic of the composition. The aqueous carrier may, for example, be water or water solutions of lower alkyl alcohols or polyhydric alcohols. The lower alkyl alcohols may, for example, be monohydric alcohols having 1 to 6 carbons, often ethanol and/or isopropanol. The polyhydric alcohols may, for example, be propylene glycol, hexylene glycol, glycerin, and/or propane diol. Preferably, the aqueous carrier is substantially water. Deionized water is preferably used. Water from natural sources, including minerals can also be used, depending on the desired characteristic of the composition. Generally, the composition of the invention can comprise up to 80 %, often even up to 95 % by weight of water.

The compositions of the invention may also include as a further component (F), other components being suitable for rendering the compositions more cosmetically or aesthetically acceptable or to provide them with additional usage benefits. Such other components can generally be used individually at levels of from 0.001 % to 5 % by weight. A wide variety of further components (F) can be formulated into the composition of the invention. These include conditioning agents, such as hydrolysed collagen, vitamin E, panthenol, panthenyl ethyl ether, hydrolysed keratin, proteins, plant extracts, nutrients; emollients, such as PPG-3 myristyl ether, trimethyl pentanol hydroxyethyl ether; hair-fixative polymers, such as amphoteric fixative polymers, cationic fixative polymers, anionic fixative polymers, nonionic fixative polymers, silicone grafted copolymers; preservatives, such as benzyl alcohol, methyl paraben, propyl paraben, imidazolidinyl urea; pH adjusting agents, such as citric acid, sodium citrate, succinic acid, phosphoric acid, sodium hydroxide, sodium carbonate; salts, in general, such as potassium acetate or sodium chloride; coloring agents; hair oxidizing (bleaching) agents, such as hydrogen peroxide, perborate or persulfate salts; hair reducing agents such as thioglycolates; perfumes; sequestering agents, such as disodium ethylene-diamine tetraacetate; ultraviolet and infrared screening and absorbing

agents, such as octyl salicylate; anti-dandruff agents, such as zinc pyrithione, piroctone olamine or salicylic acid.

In at least one embodiment, the composition of the invention comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, zinc pyrithione, piroctone olamine (octopirox), and combinations thereof. In at least one embodiment, the composition of the invention comprises a total amount of anti-fungal substance in the composition of from 0.1 wt.-% to 1 wt.-%. In at least one embodiment, the composition of the invention comprises piroctone olamine. In at least one embodiment, the composition of the invention comprises a pyridinethione anti-dandruff particulate. For example, 1-hydroxy-2-pyridinethione salts are highly preferred particulate anti-dandruff agents. The concentration of pyridinethione anti-dandruff particulate may range from 0.1% to 4% by weight of the formulation, preferably from 0.1% to 3%, more preferably from 0.3% to 2%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982. It is contemplated that when ZPT is used as the anti-dandruff particulate in the compositions herein, that the growth or regrowth of hair may be stimulated or regulated, or both, or that hair loss may be reduced or inhibited, or that hair may appear thicker or fuller.

Preferably, salt is present at levels from 0.1 to 1 wt.-% of the total composition to adjust the product viscosity. Preferably, NaOH is present at levels from 0.1 to 1 wt.-% of the total composition to adjust the pH of the formulation.

The composition of the invention may contain as a further component (F) a polysorbate for adjusting rheology, for example, polysorbate-20, polysorbate-21, polysorbate-40, polysorbate-60, or mixtures thereof. The polysorbate can be contained in the composition in amounts up to 5% (e.g. 0.1 to 5%) by weight.

The composition of the invention can also contain as a further component (F) a polypropylene glycol. Preferred polypropylene glycols are those having a weight average molecular weight of from 200 to 100000 g/mol. The polypropylene glycol may be either water-soluble, water-insoluble, or may have a limited solubility in water, depending upon the degree of polymerization and whether other moieties are attached thereto. The desired solubility of the polypropylene glycol in water will depend in large part upon the form of the composition (e.g., leave-on hair care composition, rinse-off hair care composition). The polypropylene glycol can be included in the composition of the invention at a level of up to 10% by weight.

For example, in a rinse-off hair care composition, it is preferred that the polypropylene glycol has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the composition of the invention at a level of up to 10% by weight.

The composition of the invention can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, or glyceryl ester oils; poly [alpha]-olefin oils; and mixtures thereof. Preferred low melting point oils are selected from the group consisting of ester oils such as pentaerythritol ester oils, trimethylol ester oils, citrate ester oils, or glyceryl ester oils; poly [alpha]-olefin oils; and mixtures thereof. Particularly useful pentaerythritol ester oils and trimethylol ester oils are pentaerythritol tetraisostearate, pentaerythritol tetraoleate, trimethylolpropane triisostearate, trimethylolpropane trioleate, or mixtures thereof. Particularly useful glyceryl esters are triisostearin, triolein or trilinolein.

The composition of the invention can also contain, as a further component (F), a cationic polymer. Cationic polymers may be present in the composition of the invention for further enhancing deposition performance.

Suitable cationic polymers may be homopolymers which are cationically substituted or may be formed from two or more types of monomers. The weight average (Mw) molecular weight of the polymers will generally be between 100 000 and 2 million g/mol. The polymers will have cationic nitrogen containing groups such as quaternary ammonium or protonated amino groups, or a mixture thereof.

If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of aminoalkyl esters of homo- and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula: A-O-[R-$N^+$(R1)(R2)(R3)$X^-$], wherein: A is an anhydroglucose residual group, such as a starch or cellulose anhydroglucose residual. R is an alkylene, oxyalkylene, polyoxyalkylene, or hydroxyalkylene group, or combination thereof. R1, R2 and R3 independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R1, R2 and R3) is preferably about 20 or less, and $X^-$ is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer may be present in the composition of the invention at levels of from 0.01 to 5 wt.-%, preferably from 0.05 to 1 wt.-%, more preferably from 0.08 to 0.5 wt.-% by total weight of cationic polymer based on the total weight of the composition.

In at least one embodiment, the cationic polymers have a number average molecular weight of at least about 5000 g/mol, typically from 10000 g/mol to 10 million g/mol and are selected from the group consisting of copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar® from Solvay.

In at least one embodiment, the composition of the invention comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants.

In at least one embodiment, the composition of the invention comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the composition of the invention comprises an anionic surfactant. In at least one embodiment, the anionic surfactant is selected from the group consisting of (C10-C20)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the

form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1. Preferably the level of alkyl ether sulphate is from 0.5 wt.-% to 25 wt.-% of the total composition, more preferably from 3 wt.-% to 18 wt.-%, most preferably from 6 wt.-% to 15 wt.-% of the total composition.

The total amount of anionic surfactant in compositions of the invention may range from 0.5 wt.-% to 45 wt.-%, more preferably from 1.5 wt.-% to 20 wt.-%.

Compositions of the invention may comprise fatty acyl isethionate, if present preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 %. Preferably, greater than 20 wt.-% and less than 45 wt.-%, more preferably greater than 25 wt.-% and less than 45 wt.-% of the fatty acyl isethionate are of chain length greater than or equal to C16; and greater than 50 wt.-%, preferably greater than 60 wt.-% of the free fatty acid/soap is of chain length C16 to C20. In addition, the product may contain isethionate salts, which are present typically at levels less than 5 wt.-%, and traces (less than 2 wt.-%) of other impurities.

Preferably, a mixture of aliphatic fatty acids is used for the preparation of commercial fatty acyl isethionate surfactants. The resulting fatty acyl isethionate surfactants (e.g., resulting from reaction of alkali metal isethionate and aliphatic fatty acid) preferably should have more than 20 wt.-%, preferably more than 25 wt.-%, but no more than 45 wt.-%, preferably 35 % (on basis of fatty acyl isethionate reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the composition of the invention comprises an acylglycinate surfactant. In at least one embodiment, the acylglycinate surfactant conforms to the formula (Y):

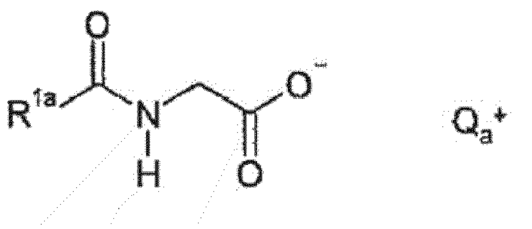

(Y)

wherein

$R^{1a}$ is a linear or branched, saturated alkyl group having 6 to 30, preferably 8 to 22, particularly preferably 8 to 18, carbon atoms or is a linear or branched, mono- or polyunsaturated alkenyl group having 6 to 30, preferably 8 to 22 and particularly preferably 12 to 18 carbon atoms, and

$Qa^+$ is a cation.

In at least one embodiment, $Q_a^+$ is selected from the group consisting of $Li^+$, $Na^+$, K+, $Mg^{++}$, $Ca^{++}$, $Al^{+++}$, $NH_4^+$, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C12 alkyl or C14 alkyl. In at least one embodiment, the acylglycinate surfactant is selected from those conforming to formula (Y), wherein R is C16 alkyl or C18 alkyl.

In at least one embodiment, the composition comprises from 0.01 wt.-% to 30 wt. %, or 1 wt.-% to 25 wt.-%, preferably from 5 wt.-% to 20 wt.-%, more preferably from 12 wt.-% to 18 wt.-% anionic surfactant.

In at least one embodiment, the composition of the invention comprises a glutamate surfactant corresponding

to formula (Z) or a salt thereof:

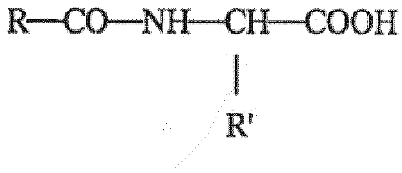

(Z)

wherein R' is HOOC-CH2-CH2- or $M^{+-}$OOC-CH2-CH2- wherein $M^+$ is a cation; and wherein R is a linear or branched, saturated alkyl group having 6 to 30, preferably 8 to 22, more preferably 8 to 18, carbon atoms or is a linear or branched, mono- or polyunsaturated alkenyl group having 6 to 30, preferably 8 to 22 and more preferably 12 to 18 carbon atoms. In at least one embodiment, $M^+$ is a metal cation. In at least one embodiment, $M^+$ is selected from the group consisting of $Li^+$, $Na^+$, $K^+$, $Mg^{++}$, $Ca^{++}$, $Al^{+++}$, $NH4^+$, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C12 alkyl or C14 alkyl. In at least one embodiment, the glutamate surfactant is selected from those conforming to formula (Z), wherein R is C16 alkyl or C18 alkyl.

In at least one embodiment, the composition of the invention comprises a non-ionic surfactant. The non-ionic surfactants may be present in the range 0 to 5 wt.-%. Nonionic surfactants that can be included in compositions of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

$$R\text{-}(OCH_2CH_2)_nOH,$$

where R is an alkyl chain of C12 to C15, and n is 5 to 9. Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

$$RO\text{-}(G)_n$$

wherein R is a branched or straight chain alkyl group which may be saturated or unsaturated and G is a saccharide group. R may represent a mean alkyl chain length of from about C5 to about C20. Preferably R represents a mean alkyl chain length of from about C9 to about C12. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the fatty (e.g. C10-C18) N-alkyl (C1-C6) polyhydroxy fatty acid amides, such as the C12-C18 N-methyl glucamides, as described for example in WO9206154 and US5194639, and the N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C8- to C22-fatty acids and a degree of ethoxylation of from 4 to 20, polyglyceryl fatty acid esters, in particular of one, two or more C8- to C22-fatty acids and polyglycerol having preferably 2 to 20 glyceryl units, alkyl glucosides, alkyl oligoglucosides and alkyl polyglucosides having C8 to C22-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics®), fatty acid alkylol amides, (fatty acid amide polyethylene glycols), N-alkyl-, N-alkoxypoly-hydroxy-fatty acid amide, sucrose esters, sorbitol esters, polyglycol ethers, and mixtures thereof.

In at least one embodiment, the composition of the invention comprises a fatty N-methyl-N-glucamide surfactant. In at least one embodiment, the fatty N-methyl-N-glucamide surfactant conforms to the formula (X):

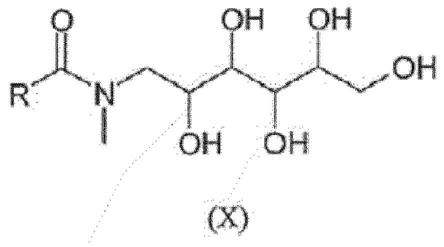

(X)

wherein

R is a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1 butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2 methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3 methyl-2-pentyl, 4-methyl-2-pentyl, 2 methyl-3-pentyl, 3-methyl-3-pentyl, 2,2 dimethyl-1-butyl, 2,3-dimethyl-1-butyl, 3,3 dimethyl-1-butyl, 2-ethyl-1-butyl, 2,3-dimethyl-2-butyl, 3,3-dimethyl-2-butyl, 1 heptyl, 1-octyl, 1-nonyl, 1-decyl, 1 undecyl, 1-dodecyl, 1-tetradecyl, 1-hexadecyl and 1-octadecyl. Suitable fatty N methyl-N-glucamide surfactants are described in WO2013/178700 and EP 0 550 637, which are incorporated herein by reference. In at least one embodiment, the N-methyl-N-glucamide surfactant is selected from those conforming to formula (X), wherein R is C12 alkyl or C14 alkyl. In at least one embodiment, the N-methyl-N-glucamide surfactant is selected from those conforming to formula (X), wherein R is C16 alkyl or C18 alkyl.

In at least one embodiment, the composition of the invention comprises from 1 wt.-% to 20 wt.-%, more preferably from 2 wt.-% to 10 wt.-%, even more preferably from 3 wt.-% to 7 wt.-% non-ionic surfactant.

Amphoteric or zwitterionic surfactant can be included in the composition of the invention in an amount ranging from 0.5 wt.-% to about 8 wt.-%, preferably from 1 wt.-% to 4 wt.-% of the total composition.

In at least one embodiment, the amphoteric surfactants are selected from the group consisting of N-($C_{12}$-$C_{18}$)-alkyl-beta-aminopropionates and N-($C_{12}$-$C_{18}$)-alkyl-beta-iminodipropionates as alkali metal salts and mono-, di-, and trialkylammonium salts; N-acylaminoalkyl-N,N-dimethylacetobetaine, preferably N-($C_8$-$C_{18}$)-acylaminopropyl-N,N-dimethylacetobetaine, ($C_{12}$-$C_{18}$)-alkyl-dimethyl-sulfopropylbetaine, amphosurfactants based on imidazoline (trade name: Miranol®, Steinapon®), preferably the sodium salt of 1-(beta-carboxymethyloxyethyl)-1-(carboxymethyl)-2-laurylimidazolinium; amine oxide, e.g., ($C_{12}$-$C_{18}$)-alkyl-dimethylamine oxide, fatty acid amidoalkyldimethylamine oxide, and mixtures thereof.

In at least one embodiment, the composition of the invention comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C8- to C18-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C8- to C18-alkyldimethylammonium acetates, the C8-to C18 alkyldimethylcarbonylmethylammonium salts, and the C8- to C18-fatty acid alkylamidobetaines, and mixtures thereof. Optionally, the C8- to C18-fatty acid alkylamidobetaine is selected from coconut fatty acid amidopropylbetaine, N-coconut fatty acid amidoethyl-N-[2-(carboxymethoxy)ethyl]glycerol (CTFA name: cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the composition of the invention comprises from 0.5 wt.-% to 20 wt.-%, preferably from 1 wt.-% to 10 wt.-% amphoteric surfactant.

In at least one embodiment, the composition of the invention comprises a surfactant system. In at least one embodiment, the surfactant system comprises at least one surfactant selected from the group consisting of lauryl sulfate, laureth sulfate, cocamidopropyl betaine, sodium cocoylglutamate, lauroamphoacetate, and mixtures thereof. In at least one embodiment, the surfactant system comprises sodium laureth sulfate, sodium lauryl sulfate, and optionally cocamidopropyl betaine. In at least one embodiment, the surfactant system comprises sodium laureth sulfate, potassium cocoylglutamate, and cocamidopropyl betaine.

In at least one embodiment, the composition of the invention contains as a further component a silicone compound. The composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, nonionic surfactants, cationic surfactants, and mixtures thereof.

In at least one embodiment, the composition of the invention contains silicone conditioning agents. Preferably, these are emulsified droplets of a silicone conditioning agent. These are for enhancing conditioning performance.

Suitable silicones include polydiorganosiloxanes, in particular polydimethylsiloxanes, which have the CTFA designation dimethicone. Also suitable for use in the compositions of the invention (particularly shampoos and hair conditioners) are polydimethyl siloxanes having hydroxyl end groups, which have the CTFA designation dimethiconol. Also suitable for use in the compositions of the invention (particularly shampoos and hair conditioners) are silicone gums having a slight degree of cross-linking, as are described for example in WO 96/31188. The viscosity of the emulsified silicone itself (not the emulsion or the final composition) is typically at least 10,000 cSt at 25°C. The viscosity of the silicone itself is preferably at least 60,000 cSt, most preferably at least 500,000 cSt, ideally at least 1,000,000 cSt. Preferably, the viscosity does not exceed $1x10^9$ cSt for ease of formulation. Emulsified silicones for use in the compositions of the invention (particularly shampoos) will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in the compositions of the invention (particularly shampoos and hair conditioners) are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone".

Specific examples of amino functional silicones suitable for use in the compositions of the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactant. Pre-formed emulsions of amino functional silicones are also available from suppliers of silicone oils such as Dow Corning and General Electric.

Specific examples include DC939 Cationic Emulsion and the non-ionic emulsions DC2-7224, DC2- 8467, DC2-8177 and DC2-8154 (all ex Dow Corning).

Combination of amino and non-amino functional silicones may also be used.

The total amount of silicone is preferably from 0.01 wt.-% to 10 wt.-% of the total composition, more preferably from 0.1 wt.-% to 5 wt.-%, most preferably from 0.5 wt.-% to 3 wt.-%.

In at least one embodiment, the composition of the invention comprises a preservative or preservative system. Examples of suitable preservatives include benzyl alcohol, piroctone olamine, phenoxyethanol, parabens, pentanediol, benzoic acid/sodium benzoate, sorbic acid/potassium sorbate, and other organic acids used to provide antimicrobial protection. Preservation boosting ingredients include anisic acid, lactic acid, sorbitan caprylate, ethylhexylglycerin, caprylyl glycol, octanediol, and similar substances. In at least one embodiment, the composition comprises 0.01 to 5 wt.-%,

particularly preferably from 0.05 to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethylsarcosinate, lauroylsarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, Octopirox®, methylchloroisothiazolinone, methylisothiazolinone, methyldibromo glutaronitrile, AgCI, chloroxylenol, sodium salts of diethylhexylsulfosuccinate, sodiumbenzoate, phenoxyethanol, benzylalkohol, phenoxyisopropanol, paraben, such as butyl-, ethyl-, methyl- und propylparaben, and their salts, pentandiol, 1,2-octanediol, ethylhexylglycerinw, benzylalcohol, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition is substantially free of parabens.

The composition of the invention may also comprise a dispersed, non-volatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C8-C22 carboxylic acids. Preferred materials include cocoa butter, palm stearin, sunflower oil, soybean oil and coconut oil.

The oily or fatty material may be present at a level of from 0.05 to 10 wt.-%, preferably from 0.2 to 5 wt.-%, more preferably from 0.5 to 3 wt.-%, based on the total weight of the composition.

In a particularly preferred embodiment, the composition of the invention is a shampoo composition.

In at least one embodiment, the shampoo composition comprises from 1 to 99%, preferably from 5 to 95%, more preferably from 10 to 90% by weight of the total composition of water, and from 0.1 to 99%, preferably from 1 to 95%, more preferably from 5 to 90%, often 5 to 25% by weight of the total composition of a cleansing surfactant. Suitable cleansing surfactants are generally anionic, amphoteric, betaine, or zwitterionic surfactants. For example, the anionic surfactants are alkyl ether or alkyl ether sulfates, such as sodium lauryl sulfate, or other compounds described above.

In at least one embodiment, the shampoo composition comprises one or more further cosmetically acceptable components (F), which can be present in an amount of at least 0.5% by weight, or from 0.5 to 20% by weight, by total weight of the shampoo composition. Preferably, the component (F) is selected from the group consisting of cleansing ingredients, acidity regulators, colorants, conditioning agents, emulsifiers, film formers, fragrances, glossers, humectants, lubricants, moisturizers, pigments, preservatives, hair penetration enhancers, scalp actives, stabilizers, surfactants, thickeners, viscosity modifiers, and combinations thereof. More preferably, the component (F) is selected from the group consisting of surfactants, viscosity-modifying polymers and conditioning ingredients. In at least one embodiment, the shampoo composition comprises from 0.3 to 10% by weight of the multilamellar vesicles described herein and further comprises at least 0.5% by weight of one or more further components (F) selected from the group consisting of surfactants, polymers, conditioning agents, actives, acidity regulators, lubricants, moisturisers, oils, preservatives, sequestrants,

strengtheners, sun protectors, and combinations thereof.

In at least one embodiment, the shampoo composition comprises further cosmetically acceptable components (F) being cleansing ingredients. In at least one embodiment, the shampoo composition comprises from 0.05% to 20% cleansing ingredients. In at least one embodiment of the shampoo composition, the level of cleansing ingredient is from 1% to 20% by total weight of the active ingredient in the composition, preferably 5% to 18%, more preferably 8% to 16%. In at least one embodiment, the cleansing ingredient is selected from the group consisting of non-polymeric surfactants, saponins, polymeric surfactants, and combinations thereof. Preferably the cleansing ingredient comprises or consists of surfactants.

In at least one embodiment, the shampoo composition comprises from 0.3 to 10 % by weight of the multilamellar vesicles described herein and at least 0.5 % by weight of surfactants, preferably cleansing anionic or nonionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the shampoo composition is silicone-free. In at least one embodiment, the shampoo composition is sulfate-free. In at least one embodiment, the shampoo composition is silicone-free and sulfate-free.

In at least one embodiment, the shampoo composition comprises:

(i) from 0.3 wt.-% to 10 wt.-% of the multilamellar vesicles described herein;
(ii) from 5 wt.-% to 20 wt.-% of one or more anionic surfactants;
(iii) at least 50 wt.-% water; and
(iv) at least one further cosmetically acceptable component (F) selected from the group consisting of silicone, cationic polymer, rheology modifiying agent, and an amphoteric or zwitterionic surfactant.

In at least one embodiment, the shampoo composition comprises:

(i) from 0.3 wt.-% to 10 wt.-% of the multilamellar vesicles described herein;
(ii) from 5 wt.-% to 20 wt.-% of one or more anionic surfactants;
(iii) at least 50 wt.-% water; and
(iv) at least one further component selected from the group consisting of silicone, cationic polymer, rheology modifiying agent, and an amphoteric or zwitterionic surfactant;
(v) at least one further cosmetically acceptable component (F).

In at least one embodiment, the shampoo composition consists of:

(i) from 0.3 wt.-% to 10 wt.-% of the multilamellar vesicles described herein;
(ii) from 5 wt.-% to 20 wt.-% of one or more anionic surfactants;
(iii) at least 50 wt.-% water; and
(iv) at least one further component selected from the group consisting of silicone, cationic polymer, rheology modifiying agent, and an amphoteric or zwitterionic surfactant;
(v) at least one further cosmetically acceptable component (F) selected from the group consisting of conditioning agents, such as hydrolysed collagen, vitamin E, or panthenol, panthenyl ethyl ether, hydrolysed keratin, proteins, plant extracts, nutrients; and emollients such as PPG-3 myristyl ether, trimethyl pentanol hydroxyethyl ether; hairfixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents such as the thioglycolates; perfumes; and sequestering agents, such as disodium ethylene-diamine tetraacetate; ultraviolet and infrared screening and absorbing agents such as octyl salicylate; and anti-dandruff agents such as zinc pyrithione, piroctone olamine and salicylic acid.

The composition of the invention may comprise as component (F) a cationic softening agent. If present, the cationic softening agent is usually present in the compositions of the invention at a level of 2 to 75% by weight, preferably 10 to 50% by weight, more preferably 20 to 35% by weight of the total composition. Also preferably, the level of the cationic softening agent is 10 to 30% by weight of the total composition. Also preferably, the level of the cationic softening agent is 0.5 to 8% by weight of the total composition.

The cationic softening agent is preferably one that is able to form a lamellar phase dispersion in water, in

particular a dispersion of liposomes.

The cationic softening agent is preferably a quaternary ammonium compound, in particular one having two $C_{12-28}$ groups connected to the nitrogen head group, preferably being connected to the nitrogen head group by at least one ester link, and more preferably by two ester links. The $C_{12-28}$ groups may independently be alkyl or alkenyl groups.

The average chain length of the alkyl and/or alkenyl groups is preferably at least C14 and more preferably at least C16. It is particularly preferred that at least half of the groups have a chain length of C18. In general, the alkyl and/or alkenyl groups are predominantly linear.

The cationic softening agent is preferably an ester-linked quaternary ammonium compound. Preferably, the quaternary ammonium compound has fatty acid chains. The fatty acid chains of the quaternary ammonium compound preferably comprise from 10 to 50 wt%, preferably from 20 to 35 wt% of saturated C18 chains and from 10 to 50 wt%, preferably from 20 to 35 wt% of monounsaturated C18 chains by weight of total fatty acid chains.

Preferably, the quaternary ammonium compound is derived from palm or tallow feedstocks. These feedstocks may be pure or predominantly palm or tallow based. Blends of different feedstocks may be used.

In a preferred embodiment, the fatty acid chains of the quaternary ammonium compound comprise from 25 to 30 wt%, preferably from 26 to 28 wt% of saturated C18 chains and from 25 to 30 wt%, preferably from 26 to 28 wt% of monounsaturated C18 chains, by weight of total fatty acid chains.

In another preferred embodiment, the fatty acid chains of the quaternary ammonium compound comprise from 30 to 35 wt%, preferably from 33 to 35 wt% of saturated C18 chains and from 24 to 35 wt%, preferably from 27 to 32 wt% of monounsaturated C18 chains, by weight of total fatty acid chains.

The cationic softening agent is preferably an ester-linked triethanolamine (TEA) based quaternary ammonium compound. Ester-linked TEA based quaternary ammonium compounds preferably comprise a mixture of mono-, di- and tri-ester linked components. The tri-ester content is preferably below 20 wt %, more preferably from 5 to 9 wt % by total weight of the quaternary active component. Preferred ester-linked TEA based quaternary ammonium compounds have a diester content of from 40 to 65 wt %, more preferably from 52 to 59 wt % by total weight of the quaternary active component. Also preferred are ester-linked TEA based quaternary ammonium compounds having a monoester content of from 30 to 45 wt %, more preferably from 32 to 42 wt % by total weight of the quaternary active component. A preferred ester-linked TEA based quaternary ammonium compound comprises from 32 to 42 wt % of monoester, from 52 to 59 wt % of diester and from 5 to 9 wt % of triester compounds, by total weight of the quaternary active; more preferably from 35 to 39 wt % of monoester, from 54 to 58 wt % of diester and from 7 to 8 wt % of triester compounds, by total weight of the quaternary active component.

The quaternary ammonium compounds are also known as "soft" materials. Iodine value as used in the context of the present invention refers to the measurement of the degree of unsaturation present in a material by a method of NMR spectroscopy as described in Anal. Chem., 34, 1136 (1962), Johnson and Shoolery. In preferred embodiments, the quaternary ammonium compounds are derived from feedstock having an overall iodine value of from 30 to 60, more preferably from 30 to 45 and most preferably from 32 to 38.

A first group of quaternary ammonium compounds suitable for use in the present invention is represented by formula (I):

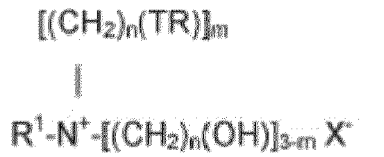

(I)

wherein each R is independently selected from a $C_{5-35}$ alkyl or alkenyl group; $R^1$ represents a $C_{1-4}$ alkyl, $C_{2-4}$ alkenyl or a $C_{1-4}$ hydroxyalkyl group; T is generally O-CO (i.e. an ester group bound to R via its carbon atom), but may alternatively be CO-O (i.e. an ester group bound to R via its oxygen atom); n is a number selected from 1 to 4; m is a number selected from 1, 2, or 3; and $X^-$ is an anionic counterion, such as a halide or alkyl sulphate, e.g. chloride or methylsulphate. Di-ester variants of formula I (i.e. m = 2) are preferred and typically have mono- and tri-ester analogues associated with them. Such materials are particularly suitable for use in the present invention.

Especially preferred agents are di-esters of triethanolammonium methylsulphate. Commercial examples include Prapagen TQ range, ex Clariant, and Tetranyl brand, ex Kao, (both di[hardened tallow ester] of triethanolammonium methylsulphate), and Rewoquat WE products, ex Evonik.

The second group of quaternary ammonium compounds suitable for use in the invention is represented by formula (II):

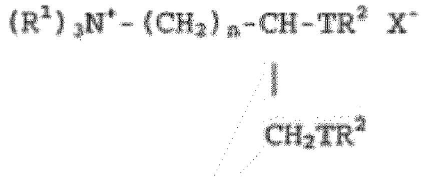

(II)

wherein each $R^1$ group is independently selected from $C_{1-4}$ alkyl, hydroxyalkyl or $C_{2-4}$ alkenyl groups; and wherein each $R^2$ group is independently selected from $C_{8-28}$ alkyl or alkenyl groups; and wherein n, T, and $X^-$ are as defined above.

Preferred materials of this second group include 1,2 bis[tallowoyloxy]-3-trimethylammonium propane chloride, 1,2 bis[hardened tallowoyloxy]-3-trimethylammonium propane chloride, 1,2-bis[oleoyloxy]-3-trimethylammonium propane chloride, and 1,2 bis[stearoyloxy]-3-trimethylammonium propane chloride. Such materials are described in US 4,137,180 (Lever Brothers). Preferably, these materials also comprise an amount of the corresponding mono-ester.

A third group of quaternary ammonium compounds suitable for use in the invention is represented by formula (III):

$$(R^1)_2\text{-}N^+\text{-}[(CH_2)_n\text{-}T\text{-}R^2]_2\ X^- \qquad \text{(III)}$$

wherein each $R^1$ group is independently selected from $C_{1-4}$ alkyl, or $C_{2-4}$ alkenyl groups; and wherein each $R^2$ group is independently selected from $C_{8-28}$ alkyl or alkenyl groups; and n, T, and $X^-$ are as defined above. Preferred materials of this third group include bis(2-tallowoyloxyethyl)dimethyl ammonium chloride and hardened versions thereof.

A fourth group of quaternary ammonium compounds suitable for use in the invention is represented by formula (IV):

$$(R_1)_2\text{-}N^+\text{-}(R^2)2\ X^- \qquad \text{(IV)}$$

wherein each $R^1$ group is independently selected from $C_{1-4}$ alkyl, or $C_{2-4}$ alkenyl groups; and wherein each $R^2$ group is independently selected from $C_{8-28}$ alkyl or alkenyl groups; and $X^-$ is as defined above. Preferred materials of this fourth group include di(hardened tallow)dimethylammonium chloride.

Further quaternary ammonium compounds are described further above.

The composition of the invention may comprise as component (F) a hydrophobic agent. If present, the hydrophobic agent is usually present in an amount of from 0.05 to 1.0 wt %, preferably from 0.1 to 0.8 wt %, more preferably from 0.2 to 0.7 wt % and most preferably from 0.4 to 0.7 wt% by weight of the total composition, for example from 0.2 to 0.5 wt % by weight of the total composition.

Suitable hydrophobic agents include esters derived from the reaction of a fatty acid with an alcohol. The fatty acid preferably has a carbon chain length of from C8 to C22 and may be saturated or unsaturated, preferably saturated. Some examples include stearic acid, palmitic acid, lauric acid and myristic acid. The alcohol may be linear, branched or cyclic. Linear or branched alcohols have a preferred carbon chain length of from 1 to 6. Preferred alcohols include methanol, ethanol, propanol, isopropanol, sorbitol. Preferred hydrophobic agents include methyl esters, ethyl esters, propyl esters, isopropyl esters and sorbitan esters derived from such fatty acids and alcohols.

Non-limiting examples of suitable hydrophobic agents include methyl esters derived from fatty acids having a carbon chain length of from at least C10, ethyl esters derived from fatty acids having a carbon chain length of from at least C10, propyl esters derived from fatty acids having a carbon chain length of from at least C8, isopropyl esters derived from fatty acids having a carbon chain length of from at least C8, sorbitan esters derived from fatty acids having a carbon chain length of from at least C16, and alcohols with a carbon chain length greater than C10. Naturally occurring fatty acids commonly have a carbon chain length of up to C22.

Some preferred materials include methyl undecanoate, ethyl decanoate, propyl octanoate, isopropyl myristate, sorbitan stearate and 2-methyl undecanol, ethyl myristate, methyl myristate, methyllaurate, isopropyl palmitate and ethyl stearate; more preferably methyl undecanoate, ethyl decanoate, isopropyl myristate, sorbitan stearate, 2-methyl undecanol, ethyl myristate, methyl myristate, methyl laurate and isopropyl palmitate.

Non-limiting examples of such materials include methyl undecanoate, ethyl decanoate, propyl octanoate, isopropyl myristate, sorbitan stearate and 2-methyl undecanol; preferably methyl undecanoate, ethyl decanoate, isopropyl myristate, sorbitan stearate and 2-methyl undecanol. Most preferably, the hydrophobic agent is isopropyl myristate.

The composition of the invention may comprise as component (F) a non-ionic alkoxylated material. The addition of such a non-ionic material reduces the occurrence of flocculation when the composition is added to water, such as rinse water. If present, the non-ionic alkoxylated material may be present in an amount of from 0.01 to 0.5 wt %, preferably from 0.02 to 0.4 wt %, more preferably from 0.05 to 0.25 wt% and most preferably 0.1 wt % by total weight of the composition.

Suitable non-ionic alkoxylated materials include non-ionic surfactants. Suitable non-ionic surfactants include

addition products of ethylene oxide and/or propylene oxide with fatty alcohols, fatty acids and fatty amines. The non-ionic alkoxylated material is preferably selected from addition products of (a) an alkoxide selected from ethylene oxide, propylene oxide and mixtures thereof with (b) a fatty material selected from fatty alcohols, fatty acids and fatty amines.

Suitable surfactants are, for example, substantially water-soluble surfactants of the general formula:

$$R\text{-}Y\text{-}(C_2H_4O)_z\text{-}CH_2\text{-}CH_2\text{-}OH$$

where R is selected from the group consisting of primary, secondary and branched chain alkyl and/or acyl hydrocarbyl groups; primary, secondary and branched chain alkenyl hydrocarbyl groups; and primary, secondary and branched chain alkenyl- substituted phenolic hydrocarbyl groups; the hydrocarbyl groups having a chain length of from 10 to 60, preferably 10 to 25, e.g. 14 to 20 carbon atoms.

In the general formula for the ethoxylated non-ionic surfactant, Y is typically:

-O-, -C(O)O-, -C(O)N(R)- or -C(O)N(R)R-

in which R has the meaning given above or can be hydrogen; and Z is at least about 6, preferably at least about 10 or 11.

Lutensol™ AT25 (BASF) based on coco chain and 25 EO groups is an example of a suitable nonionic surfactant. Other suitable surfactants include Renex 36 (Trideceth-6), ex Uniqema; Tergitoi 15-S3, ex Dow Chemical Co.; Dihydrol LT7, ex Thai Ethoxylate ltd; Cremophor CO40, ex BASF and Neodol 91-8, ex Shell.

Further non-ionic surfactants are described further above.

The composition of the invention may comprise as component (F) a polymeric thickening agent, also referred to as thickening polymer. Thickening polymers may be added to the compositions of the invention for further thickening. Any suitable thickener polymer may be used. If present, the amount of thickening polymer used in the compositions of the invention is typically from 0.001 to 0.5 wt %, preferably from 0.005 to 0.4 wt %, more preferably from 0.05 to 0.35 wt% and most preferably from 0.1 to 0.25 wt %, by weight of the total composition.

Suitable polymers are water soluble or dispersable. A high M.Wt, (for example, in the region of about 100,000 to 5,000,000), which can be achieved by crosslinking, is advantageous. Preferably, the polymer is cationic.

Polymers particularly useful in the compositions of the invention include those described in WO 2010/078959 (SNF S.A.S.). These are crosslinked water-swellable cationic copolymers having at least one cationic monomer and optionally other non-ionic and/or anionic monomers. Preferred polymers of this type are copolymers of acrylamide and trimethylaminoethylacrylate chloride.

Preferred polymers comprise less than 25 % of water-soluble polymers by weight of the total polymer, preferably less than 20 %, and most preferably less than 15 %, and a cross-linking agent concentration of from 500 ppm to 5000 ppm relative to the polymer, preferably from 750 ppm to 5000 ppm, more preferably from 1000 to 4500 ppm (as determined by a suitable metering method such as that described on page 8 of patent EP 343840). The cross-linking agent concentration must be higher than about 500 ppm relative to the polymer and preferably higher than about 750 ppm when the crosslinking agent used is the methylene bisacrylamide, or other cross-linking agents at concentrations that lead to equivalent cross-linking levels of from 10 to 10,000 ppm.

Suitable cationic monomers are selected from the group consisting of the following monomers and derivatives and their quaternary or acid salts: dimethylaminopropylmethacrylamide, dimethylaminopropylacrylamide, diallylamine, methyldiallylamine, dialkylaminoalkyl-acrylates and methacrylates, dialkylaminoalkyl-acrylamides or -methacrylamides.

Following is a non-restrictive list of monomers performing a non-ionic function: acrylamide, methacrylamide, N-alkyl acrylamide, N-vinyl pyrrolidone, N-vinyl formamide, N-vinyl acetamide, vinylacetate, vinyl alcohol, acrylate esters, allyl alcohol.

Following is a non-restrictive list of monomers performing an anionic function: acrylic acid, methacrylic acid, itaconic acid, crotonic acid, maleic acid, fumaric acid, as well as monomers performing a sulfonic acid or phosphonic acid functions, such as 2-acrylamido-2-methyl propane sulfonic acid (ATBS) etc..

The monomers may also contain hydrophobic groups.

Following is a non-restrictive list of cross-linking agents: methylene bisacrylamide (MBA), ethylene glycol diacrylate, polyethylene glycol dimethacrylate, diacrylamide, triallylamine, cyanomethylacrylate, vinyl oxyethylacrylate or methacrylate and formaldehyde, glyoxal, compounds of the glycidyl ether type such as ethyleneglycol diglycidyl ether, or the epoxides or any other means familiar to the expert permitting cross-linking.

By way of preeminent preference, the cross-linking rate preferably ranges from 800 to 5000 ppm (on the basis of methylene bisacrylamide) relative to the polymer or equivalent cross-linking with a cross-linking agent of different efficiency.

As described in US 2002/0132749 and Research Disclosure 429116, the degree of non-linearity can additionally be controlled by the inclusion of chain transfer agents (such as isopropyl alcohol, sodium hypophosphite, mercaptoethanol) in the polymerisation mixture in order to control the polymeric chain's length and the cross-linking density.

An example of a preferred polymer is Flosoft 270LS ex SNF.

Further polymeric thickening agents or thickening polymers are described further above.

The composition of the invention may comprise as component (F) a non-cationic softening material, which is preferably an oily sugar derivative. An oily sugar derivative is a liquid or soft solid derivative of a cyclic polyol (CPE) or of a reduced saccharide (RSE), said derivative resulting from 35 to 100% of the hydroxyl groups in said polyol or in said saccharide being esterified or etherified. The derivative has two or more ester or ether groups independently attached to a C8-C22 alkyl or alkenyl chain.

Advantageously, the CPE or RSE does not have any substantial crystalline character at 20°C. Instead it is preferably in a liquid or soft solid state at 20°C.

The liquid or soft solid CPEs or RSEs suitable for use in the present invention result from 35 to 100% of the hydroxyl groups of the starting cyclic polyol or reduced saccharide being esterified or etherified with groups such that the CPEs or RSEs are in the required liquid or soft solid state. These groups typically contain unsaturation, branching or mixed chain lengths.

Typically, the CPEs or RSEs have 3 or more ester or ether groups or mixtures thereof, for example 3 to 8, especially 3 to 5. It is preferred if two or more of the ester or ether groups of the CPE or RSE are independently of one another attached to a C8-C22 alkyl or alkenyl chain. The C8-C22 alkyl or alkenyl groups may be branched or linear carbon chains. Preferably, 35-85% of the hydroxyl groups, most preferably 40-80%, even more preferably 45-75%, such as 45-70% are esterified or etherified. Preferably, the CPE or RSE contains at least 35% tri or higher esters, e.g. at least 40%.

The CPE or RSE has at least one of the chains independently attached to the ester or ether groups having at least one unsaturated bond. This provides a cost-effective way of making the CPE or RSE a liquid or a soft solid. It is preferred if predominantly unsaturated fatty chains, derived from, for example, rape oil, cotton seed oil, soybean oil, oleic, tallow, palmitoleic, linoleic, erucic or other sources of unsaturated vegetable fatty acids, are attached to the ester/ether groups.

These chains are referred to below as the ester or ether chains (of the CPE or RSE).

The ester or ether chains of the CPE or RSE are preferably predominantly unsaturated. Preferred CPEs or RSEs include sucrose tetratallowate, sucrose tetrarapeate, sucrose tetraoleate, sucrose tetraesters of soybean oil or cotton seed oil, cellobiose tetraoleate, sucrose trioleate, sucrose triapeate, sucrose pentaoleate, sucrose pentarapeate, sucrose hexaoleate, sucrose hexarapeate, sucrose triesters, pentaesters and hexaesters of soybean oil or cotton seed oil, glucose trioleate, glucose tetraoleate, xylose trioleate, or sucrose tetra-, tri-, penta- or hexa-esters with any mixture of predominantly unsaturated fatty acid chains.

The most preferred CPEs or RSEs are those with monosaturated fatty acid chains, i.e. where any polyunsaturation has been removed by partial hydrogenation. However, some CPEs or RSEs based on polyunsaturated fatty acid chains, e.g. sucrose tetralinoleate, may be used provided most of the polyunsaturation has been removed by partial hydrogenation.

The most highly preferred liquid CPEs or RSEs are any of the above but where the polyunsaturation has been removed through partial hydrogenation.

Preferably 40% or more of the fatty acid chains contain an unsaturated bond, more preferably 50% or more, most preferably 60% or more. In most cases 65% to 100%, e.g. 65% to 95% contain an unsaturated bond.

CPEs are preferred for use with the present invention. Inositol is a preferred example of a cyclic polyol. Inositol derivatives are especially preferred.

In the context of the present invention, the term cyclic polyol encompasses all forms of saccharides. Indeed, saccharides are especially preferred for use with this invention. Examples of preferred saccharides for the CPEs or RSEs to be derived from are monosaccharides and disaccharides.

Examples of monosaccharides include xylose, arabinose, galactose, fructose, sorbose and glucose. Glucose is especially preferred. Examples of disaccharides include maltose, lactose, cellobiose and sucrose. Sucrose is especially preferred. An example of a reduced saccharide is sorbitan.

The liquid or soft solid CPEs can be prepared by methods well known to those skilled in the art. These include acylation of the cyclic polyol or reduced saccharide with an acid chloride; trans-esterification of the cyclic polyol or reduced saccharide fatty acid esters using a variety of catalysts; acylation of the cyclic polyol or reduced saccharide with an acid anhydride and acylation of the cyclic polyol or reduced saccharide with a fatty acid. See for instance US 4 386 213 and AU 14416/88 (both P&G).

It is preferred if the CPE or RSE has 3 or more, preferably 4 or more ester or ether groups. If the CPE is a disaccharide, it is preferred if the disaccharide has 3 or more ester or ether groups. Particularly preferred CPEs are esters with a degree of esterification of 3 to 5, for example, sucrose tri, tetra and penta esters.

Where the cyclic polyol is a reducing sugar, it is advantageous if each ring of the CPE has one ether or ester group, preferably at the C1 position. Suitable examples of such compounds include methyl glucose derivatives.

Examples of suitable CPEs include esters of alkyl(poly)glucosides, in particular alkyl glucoside esters having

a degree of polymerisation from 1 to 2.

The length of the unsaturated (and saturated if present) chains in the CPE or RSE is C8-C22, preferably C12-C22. It is possible to include one or more chains of C1-C8.

The liquid or soft solid CPEs or RSEs which are suitable for use in the present invention are characterised as materials having a solid:liquid ratio of between 50:50 and 0:100 at 20°C as determined by T2 relaxation time NMR, preferably between 43:57 and 0:100, most preferably between 40:60 and 0:100, such as, 20:80 and 0:100. The T2 NMR relaxation time is commonly used for characterising solid: liquid ratios in soft solid products such as fats and margarines. For the purpose of the present invention, any component of the signal with a T2 of less than 100 $\mu$s is considered to be a solid component and any component with T2 $\geq$ 100 $\mu$s is considered to be a liquid component.

For the CPEs and RSEs, the prefixes (e.g. tetra and penta) only indicate the average degrees of esterification. The compounds exist as a mixture of materials ranging from the monoester to the fully esterified ester. It is the average degree of esterification which is used herein to define the CPEs and RSEs.

If present, the CPE or RSE is preferably present in the composition in an amount of 0.5-50% by weight, based upon the total weight of the composition, more preferably 1-30% by weight, such as 2-25%, e.g. 2-20%.

Particularly preferred CPEs and RSEs for use in the compositions of the invention include sucrose tetraoleate, sucrose pentaerucate, sucrose tetraerucate and sucrose pentaoleate.

The composition of the invention may comprise as component (F) a co-softener. When employed, they are typically present at from 0.1 to 20% and particularly at from 0.5 to 10%, based on the total weight of the composition.

Suitable co-softeners include fatty acids. Preferred co-softeners include fatty esters and fatty N-oxides. Fatty esters that may be employed include fatty monoesters, such as glycerol monostearate, fatty sugar esters, such as those disclosed WO 01/46361 (Unilever).

Preferred fatty acids include hardened tallow fatty acid (available under the tradename Pristerene™, ex Uniqema). Preferred fatty alcohols include hardened tallow alcohol (available under the tradenames Stenol™ and Hydrenol™, ex Cognis and Laurex™ CS, ex Albright and Wilson).

The composition of the invention may comprise as component (F) a fatty complexing agent. Especially suitable fatty complexing agents include fatty alcohols. Fatty complexing material may be used to improve the viscosity profile of the composition.

If present, the fatty complexing agent is preferably present in an amount greater than 0.3 to 5% by weight based on the total weight of the composition. More preferably, the fatty component is present in an amount of from 0.4 to 4%. The weight ratio of the mono-ester component of the quaternary ammonium fabric softening material to the fatty complexing agent is preferably from 5:1 to 1:5, more preferably 4:1 to 1:4, most preferably 3:1 to 1:3, e.g. 2:1 to 1:2.

The composition of the invention may comprise as component (F) shading dyes. Examples of suitable shading dyes are disclosed in WO 2012/072368, on pages 17 to 22. The level of shading dye present in the compositions of the invention, if present, depends on the type of shading dye. Preferred overall ranges, suitable for the present invention are from 0.00001 to 0.1 wt %, more preferably 0.0001 to 0.01 wt %, most preferably 0.0005 to 0.005 wt% by weight of the total composition.

In addition to the fragrance comprised in the multilamellar vesicles described herein, the composition of the invention may comprise additional fragrances and perfumes. Examples of suitable fragrances and perfumes are described further above. Examples of suitable fragrances and perfumes are also disclosed in WO 2012/072368, on pages 22 to 26.

The composition of the invention may comprise one or more further ingredients. Such optional further ingredients include preservatives (e.g. bactericides), pH buffering agents, perfume carriers, hydrotropes, anti-redeposition agents, soil-release agents, polyelectrolytes, anti-shrinking agents, anti-wrinkle agents, antioxidants, sunscreens, anti-corrosion agents, drape imparting agents, anti-static agents, ironing aids, pearlisers and/or opacifiers, natural oils/extracts, processing aids, e.g. electrolytes, hygiene agents, e.g. anti-bacterials and anti-fungals and skin benefit agents.

In a particularly preferred embodiment, the composition of the invention is a fabric conditioner composition. Typically, the fabric conditioner composition comprises a cationic softening agent. Preferred cationic softening agents are quaternary ammonium compounds.

In at least one embodiment, the fabric conditioner composition comprises a hydrophobic agent. In at least one embodiment, the fabric conditioner composition comprises a non-ionic alkoxylated material. In at least one embodiment, the fabric conditioner composition comprises a polymeric thickening agent. In at least one embodiment, the fabric conditioner composition comprises a non-ionic softener. In at least one embodiment, the fabric conditioner composition comprises a co-softener. In at least one embodiment, the fabric conditioner composition comprises a fatty complexing agent. The fabric conditioner composition may further comprise shading dyes, additional fragrances and perfumes, and/or further ingredients.

In at least one embodiment, the fabric conditioner composition comprises:

(i) from 0.3 wt.-% to 10 wt.-% of the multilamellar vesicles described herein;

(ii) from 10 wt.-% to 50 wt.-%, preferably from 20 wt.-% to 35 wt.-% of one or more cationic softening agents,

preferably quaternary ammonium compounds; and
(iii) at least 50 wt.-% water.

In at least one embodiment, the fabric conditioner composition comprises:

(i) from 0.3 wt.-% to 10 wt.-% of the multilamellar vesicles described herein;
(ii) from 10 wt.-% to 50 wt.-%, preferably from 20 wt.-% to 35 wt.-% of one or more cationic softening agents, preferably quaternary ammonium compounds;
(iii) at least 50 wt.-% water; and
(iv) at least one further component (F) selected from the group consisting of hydrophobic agents, non-ionic alkoxylated materials, polymeric thickening agents, non-ionic softeners, co-softeners, fatty complexing agents.

The invention further relates to a method of preparing the composition of the invention, in particular a cosmetic composition or a fabric conditioner composition, comprising the step of preparing the multilamellar vesicles described herein, and mixing the multilamellar vesicles with one or more further components (F) and preferably water. The compositions can be prepared by any conventional method well known in the art.
The invention further relates to a method of treating hair, comprising:

a) applying a shampoo composition and/or hair conditioner composition according to the invention onto wet hair and then
b) removing the shampoo composition and/or hair conditioner composition from the hair.

The invention further relates to the use of the multilamellar vesicles described herein in cosmetic compositions. Preferred cosmetic compositions are described further above. Preferred components are described further above.
The invention further relates to the use of the multilamellar vesicles described herein in fabric conditioner compositions. Preferred components are described further above.
The invention also relates to the use of the composition of the invention as a shampoo. The invention also relates to the use of the composition of the invention as a hair conditioner. The invention also relates to the use of the composition of the invention as fabric conditioner.
The invention is illustrated in more detail by the examples below.

EXAMPLES

Example 1

phase A:

| | |
|---|---|
| 2.5 % b.wt. | ethoxylated lauryl alcohol ethoxylation degree 23 (Brij L23) |
| 2.5 % b.wt. | ethoxylated stearyl alcohol ethoxylation degree 100 (Brij S100) |
| 2.5 % b.wt. | glycerine |
| 2.5 % b.wt. | water |

phase B:

| | |
|---|---|
| 10 % b.wt. | cetyl palmitate (Cutina CP) |
| 10 % b.wt. | triglyceride of caprylic acid / capric acid (Rotefan CCT) |
| 20 % b.wt. | fragrance |

phase C:

| | |
|---|---|
| 0.05 % b.wt. | methylchloroisothiazolinone (Kathon CG) |
| 49.5 % b.wt. | water |

The ingredients of phase A and of phase B are introduced into the inlet lines of an emulsification device disclosed

in US 2013/0201785 A1. The product from this emulsification device is a composition consisting essentially of multilamellar vesicles in the shape of a sphere comprising two or more concentric lipid double layers and fragrance. The fragrance loading is more than 50 % by weight, referred to the total weight of the vesicle. The product obtained from the emulsification device is introduced under stiffing into a vessel containing phase C. An aqueous composition comprising multilamellar vesicles is formed. For example 1 the average particle size measured with Horiba LA 940 is 164 nm with standard deviation 49 nm.

Example 2

| phase A: | |
|---|---|
| 2 % b.wt. | collagen hydrolysate sodium salt modified with lauric acid (LameponS) |
| 2 % b.wt. | ethoxylated lauryl alcohol ethoxylation degree 23 (Brij L23) |
| 2 % b.wt. | ethoxylated stearyl alcohol ethoxylation degree 100 (Brij S100) |
| 2.5 % b.wt. | glycerine |
| 1.1 % b.wt. | water |

| phase B: | |
|---|---|
| 5 % b.wt. | cetyl palmitate (Cutina CP) |
| 10 % b.wt. | triglyceride of caprylic acid / capric acid (Rotefan CCT) |
| 5 % b.wt. | polyvinylacetate (Dodiflow 5599) |
| 20 % b.wt. | fragrance |

| phase C: | |
|---|---|
| 0.05 % b.wt. | methylchloroisothiazolinone (Kathon CG) |
| 49.5 % b.wt. | water |

The compositions of phases A and B are processed as in Example 1 and the obtained product is diluted with phase C as described in Example 1. For example 2 the average particle size measured with Horiba LA 940 is 173 nm with standard deviation 53 nm.

Further formation of multilamellar vesicle have been characterized with DSC (differential scanning calorimetry). The results are shown in Figure 1. The sharp endothermic peak in the DSC curve of Figure 1 confirms the transition of the vesicles from a solid gel phase state to a fluid state.

Figure 1 demonstrates that the proper selection of amphiphilic substances, such as monoester waxes, triglycerides or other non-fragrance amphiphilics transform the temperature of the phase transition into a broad temperature range. Here the transition temperature covers a range from 38 °C to 78 °C.

Encapsulated fragrance samples prepared in analogy to example 2 are diluted at 10 % in water and a cotton swatch has been exposed in these diluted samples. After absorption of the fragrance encapsulated samples on the swatch they were dried at room temperature and then the samples were evaluated for release in a microchamber at controlled temperature of 80 °C for 20 minutes. The samples desorbed at 300 °C for 10 minutes and were measured with GCMS for each day. The graph of Figure 2 shows the fragrance is releasing in controlled manner.

The curves 2, 3 and 4 of the fragrance ingredients shown in Figure 2 are mentioned below:

2 4-tert.-butyl cyclohexyl acetate (log P = 3.96)
3 alpha-isomethyl ionone (log P = 4.41)
4 Lilal (log P = 2.00)

These curves demonstrate that the release characteristics of the fragrances is not dependent upon the log P value of the fragrance.

Example 3 (with higher fragrance amount in composition)

| | |
|---|---|
| Phase A | % wt |
| ethoxylated lauryl alcohol ethoxylation degree 23 (Brij L23) | 3.50 |
| ethoxylated stearyl alcohol ethoxylation degree 100 (Brij S100) | 3.50 |
| Glycerine | 2.50 |
| Water | 4.00 |
| Phase B | |
| cetyl palmitate (Cutina CP) | 7.50 |
| triglyceride of caprylic acid / capric acid (Rotefan CCT) | 7.50 |
| polyvinylacetate (Dodiflow 5599) | 7.50 |
| fragrance | 37.50 |
| Phase C | |
| Water | 26.50 |
| Total: | 100.00 |

For example 3 the average particle size measured with Horiba LA 940 is 184 nm with standard deviation 69 nm.

Example 4 (with increase in particle size)

| | |
|---|---|
| Phase A | % wt |
| ethoxylated lauryl alcohol ethoxylation degree 23 (Brij L23) | 2.50 |
| ethoxylated stearyl alcohol ethoxylation degree 100 (Brij S100) | 2.50 |
| water | 3.00 |
| Phase B | |
| cetyl palmitate (Cutina CP) | 20.00 |
| fragrance | 20.00 |
| Phase C | |
| water | 41.50 |
| glycerine | 10.00 |
| phenoxyethanol | 0.50 |
| Total: | 100.00 |

For example 4 the average particle size measured with Horiba LA 940 is 259 nm with standard deviation 58 nm.

Example 5 (with cationic surfactant)

| | |
|---|---|
| Phase A | % wt |
| ethoxylated lauryl alcohol ethoxylation degree 23 (Brij L23) | 2.50 |
| ethoxylated stearyl alcohol ethoxylation degree 100 (Brij S100) | 2.50 |
| alkyl amines (Genamin CTAC) | 2.00 |
| glycerine | 2.50 |
| water | 4.00 |

(continued)

| | |
|---|---|
| Phase B<br>cetyl palmitate (Cutina CP)<br>triglyceride of caprylic acid / capric acid (Rotefan CCT)<br>polyvinylacetate (Dodiflow 5599)<br>fragrance | <br>7.50<br>7.50<br>7.50<br>37.50 |
| Phase C<br>water | <br>26.50 |
| Total: | 100.00 |

For example 5 the average particle size measured with Horiba LA 940 is 170 nm with standard deviation 68 nm.

Example 6 (formulation of a fabric softener)

| **Name of ingredients** | **Function** | **%** |
|---|---|---|
| Propagen TQSV-IPA (Triethanolamine Esterquat) | cationic softener | 8 |
| distilled water | vehicle | up to 100 |
| colour [Sanoline Blue AE90 /9 0.1 5)] | color | 3 |
| encapsulated fragrance nanocon sample | smell | 0.3-10% |
| Perlogen 3000 (glycol distearate, laureth-4 cocamidopropyl betaine) | sheen & shine | 1 |
| Genapol LT (PEG-150 polyglyceryl-2 tristearate and laureth-3 and dipropylene glycol) | liquid thickner | 0.5 |

Examples 8 to 51:

The compositions according to the following Examples 8 to 51 represent compositions of the invention which comprise the multilamellar vesicles described herein.

Examples 8 and 9: Fabric conditioner compositions

| **Example** | **8** | **9** |
|---|---|---|
| **Ingredient** | **wt.-%** | **wt.-%** |
| Multilamellar vesicles comprising fragrance | 1.0 | 1.0 |
| Water | to 100 | to 100 |
| Hardened tallow triethanolamine-based quaternary ammonium compound | 5.0 | 4.7 |
| Cetearyl alcohol | 0.1 | 0.4 |
| Coconut fatty alcohol ethoxylate (20 EO) (non-ionic surfactant) | 0.2 | - |
| Polyurethane polymer | 0.5 | 1.5 |

Example 10: Fabric conditioner composition

| Ingredient | wt.-% |
|---|---|
| Multilamellar vesicles comprising fragrance | 1.0 |
| Water | to 100 |
| Di(hardened tallow)dimethylammonium chloride | 3.0 |
| C16-C18 hardened tallow fatty acid | 0.4 |
| Phosphoric acid | 0.02 |
| Polyurethane polymer | 0.5 |

Example 11: Fabric conditioner composition

| Ingredient | wt.-% |
|---|---|
| Multilamellar vesicles comprising fragrance | 1.0 |
| Triethanolamine-based quaternary ammonium compound | 4.5 |
| Fatty alcohol | 0.5 |
| Thickening polymer | 0.2 |
| HCl | to pH 2.5 |
| Water | to 100 |
| Non-ionic surfactant | 0.1 |
| Isopropyl myristate | 0.1 |

Examples 12 to 16: Cosmetic compositions

(Components are given in wt%)

| | Example | | | | |
|---|---|---|---|---|---|
| | 12 | 13 | 14 | 15 | 16 |
| Composition | Liquid soap | Body wash | Wet wipe lotion | Shampoo | Shampoo |
| Multilamellar vesicles comprising fragrance | 0.5 | 0.5 | 0.2 | 0.5 | 1.5 |
| SLES | 10 | - | - | - | 9 |
| Cocamido-propylbetaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | - | - | - |
| Cocoyl Methyl Glucamide | - | 3 | 2 | - | - |
| EGDS | - | 0 | - | - | 0.7 |
| PEG-7 Glyceryl Cocoate | - | - | 1 | - | - |
| Cocoyl glucoside | - | - | - | 5 | - |
| Lauryl Glucoside | - | - | - | 5 | - |

(continued)

| | Example | | | | |
|---|---|---|---|---|---|
| | 12 | 13 | 14 | 15 | 16 |
| Acrylates Copolymer | - | - | - | 2 | - |
| Glycerine | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| Preservative + N-fatty acyl-N-methyl cyclic glucamide | - | 2 | - | 1.5 | 2 |
| N-fatty acyl-N-methyl cyclic glucamide | 1.5 | - | 1.0 | - | - |
| NaCl | 1.5 | 1.0 | 0.5 | 0.3 | 1.5 |
| Water | QSP | QSP | QSP | QSP | QSP |
| Total | 100 | 100 | 100 | 100 | 100 |

Examples 17 to 20: Cosmetic compositions

(Components are given in wt%)

| | Example | | | |
|---|---|---|---|---|
| | 17 | 18 | 19 | 20 |
| Composition | Body Lotion | Intensive hand and body lotion | Anti-ageing night cream | Baby cream |
| Multilamellar vesicles comprising fragrance | 0.7 | 0.3 | 0.5 | 0.5 |
| Ethyhexyl Stearate | 7 | - | - | - |
| Decyl Oleate | 5 | - | - | - |
| Plantasens® Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex® AVC from Clariant (Ammonium Acryloyldimethyltaurate / VP Copolymer) | 0.5 | 1 | - | - |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens® Refined Organic Babassu Butter from Clariant (Orbignya Oleifera Seed Oil) | - | 2 | - | - |
| Squalane | - | 2 | 12 | - |

(continued)

| | Example | | | |
|---|---|---|---|---|
| | 17 | 18 | 19 | 20 |
| Macadamia Ternifolia Seed Oil (and) Crambe Abyssinica Seed Oil (and) Orbignya Oleifera Seed Oil | - | 2 | - | - |
| Silcare® Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning® 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina® GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex® Velvet from Clariant (Polyacrylate Crosspolymer-11) | - | - | 0.3 | - |
| Hostacerin® SFO from Clariant (Sunflower Seed Oil Sorbitol Esters) | - | - | - | 3.5 |
| Paracera® M (Cera Microcristallina) | - | - | - | 2 |
| Beeswax | - | - | - | 1 |
| Magnesium Stearate | - | - | - | 1 |
| Talc | - | - | - | 10 |
| Zinc oxide | - | - | - | 10 |
| Allantoin (Clariant) | - | - | - | 0.3 |
| Extrapon Hamamelis | - | - | - | 2 |
| D-Panthenol | - | - | - | 2 |
| Preservative + N-fatty acyl-N-methyl cyclic glucamide | - | 2 | - | 1.5 |
| N-fatty acyl-N-methyl cyclic glucamide | 1.5 | - | 1.0 | - |
| Water | QSP | QSP | QSP | QSP |
| Total | 100 | 100 | 100 | 100 |

Examples 21 to 26: Cosmetic compositions

(Components are given in wt%)

| **Example** | **21** | **22** | **23** | **24** | **25** | **26** |
|---|---|---|---|---|---|---|
| Composition | Soap formulation | Soap bar | Body wash | Shampoo | Wash cream | Body wash |
| Multilamellar vesicles comprising fragrance | 1.5 | 0.5 | 0.3 | 0.3 | 1.5 | 1.5 |
| Sodium Lauryl Sulfate | - | - | 10 | - | - | - |
| Ammonium Lauryl Sulfate | - | - | - | 12 | - | - |
| Sodium lauryl ether sulfate (SLES) | 15 | - | 2 | - | - | - |
| Cocamido-propylbetaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerine | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| Preservative + N-fatty acyl-N-methyl cyclic glucamide | - | 2 | - | 1.5 | 1 | 0.8 |
| N-fatty acyl-N-methyl cyclic glucamide | 1.5 | - | 1.0 | - | - | - |
| NaCl | 1.5 | - | 0.5 | 0.3 | - | 2.5 |
| Water | QSP | QSP | QSP | QSP | QSP | QSP |
| Total | 100 | 100 | 100 | 100 | 100 | 100 |

Examples 27 and 28: Hair conditioner compositions

| Example | 27a | 27b | 27c | 27d | 27e | 27f |
|---|---|---|---|---|---|---|
| **Ingredient** | **wt.-%** | **wt.-%** | **wt.-%** | **wt.-%** | **wt.-%** | **wt.-%** |
| Multilamellar vesicles comprising fragrance | 1.0 | 1.0 | 1.0 | 1.0 | 1.0 | 1.0 |
| Cetearyl Alcohol | 4.0 | 4.0 | 4.0 | 4.0 | 4.0 | 4.0 |
| CTAC | 2.0 | - | - | - | - | - |
| BTAC | - | 2.0 | - | - | - | - |
| BTMS | - | - | 2.0 | - | - | - |
| Genadvance® Life | - | - | - | 2.0 | - | - |
| Genadvance® Repair | - | - | - | - | 2.0 | - |
| Genadvance® Hydra | - | - | - | - | - | 2.0 |
| Xiameter PMX-200 (dimethicone) | 0.2 | 0.2 | 0.2 | 0.2 | 0.2 | 0.2 |
| Preservative | q.s. | q.s. | q.s. | q.s. | q.s. | q.s. |
| Water | Ad. 100 | Ad. 100 | Ad. 100 | Ad. 100 | Ad. 100 | Ad. 100 |
| Genadvance® Life: Clariant, INCI = Polyquaternium-116 und Butylene Glycol<br>Genadvance® Repair: Clariant, INCI = Quaternium-98<br>Genadvance® Hydra: Clariant, INCI = Lauryl/Myristyl Polyricinoleate & Glycerin | | | | | | |

| Example | 28a | 28b | 28c | 28d | 28e | 28f |
|---|---|---|---|---|---|---|
| **Ingredient** | **wt.-%** | **wt.-%** | **wt.-%** | **wt.-%** | **wt.-%** | **wt.-%** |
| Multilamellar vesicles comprising fragrance | 1.0 | 1.0 | 1.0 | 1.0 | 1.0 | 1.0 |
| Cetearyl Alcohol | 4.0 | 4.0 | 4.0 | 4.0 | 4.0 | 4.0 |
| CTAC | 1.0 | 1.0 | - | - | - | - |
| BTAC | 1.0 | - | 1.0 | - | - | - |
| BTMS | - | 1.0 | 1.0 | - | - | - |
| Genadvance® Life | - | - | - | 1.0 | 1.0 | - |
| Genadvance® Repair | - | - | - | 1.0 | - | 1.0 |
| Genadvance® Hydra | - | - | - | - | 1.0 | 1.0 |
| Xiameter PMX-200 (dimethicone) | 0.2 | 0.2 | 0.2 | 0.2 | 0.2 | 0.2 |
| Preservative | q.s. | q.s. | q.s. | q.s. | q.s. | q.s. |
| Water | Ad. 100 | Ad. 100 | Ad. 100 | Ad. 100 | Ad. 100 | Ad. 100 |
| Genadvance® Life: Clariant, INCI = Polyquaternium-116 und Butylene Glycol<br>Genadvance® Repair: Clariant, INCI = Quaternium-98<br>Genadvance® Hydra: Clariant, INCI = Lauryl/Myristyl Polyricinoleate & Glycerin | | | | | | |

| Example | 28g | 28h | 28i | 28j | 28k | 28l |
|---|---|---|---|---|---|---|
| **Ingredient** | **wt.-%** | **wt.-%** | **wt.-%** | **wt.-%** | **wt.-%** | **wt.-%** |
| Multilamellar vesicles comprising fragrance | 1.0 | 1.0 | 1.0 | 1.0 | 1.0 | 1.0 |
| Cetearyl Alcohol | 4.0 | 4.0 | 4.0 | 4.0 | 4.0 | 4.0 |
| CTAC | 0.5 | 0.5 | 0.5 | - | - | - |

(continued)

| Example | 28g | 28h | 28i | 28j | 28k | 28l |
|---|---|---|---|---|---|---|
| **Ingredient** | **wt.-%** | **wt.-%** | **wt.-%** | **wt.-%** | **wt.-%** | **wt.-%** |
| BTAC | - | - | - | 0.5 | 0.5 | 0.5 |
| BTMS | - | - | - | - | - | - |
| Genadvance® Life | 1.5 | - | - | 1.5 | - | - |
| Genadvance® Repair | - | 1.5 | - | - | 1.5 | - |
| Genadvance® Hydra | - | - | 1.5 | - | - | 1.5 |
| Xiameter PMX-200 (dimethicone) | 0.2 | 0.2 | 0.2 | 0.2 | 0.2 | 0.2 |
| Preservative | q.s. | q.s. | q.s. | q.s. | q.s. | q.s. |
| Water | Ad. 100 | Ad. 100 | Ad. 100 | Ad. 100 | Ad. 100 | Ad. 100 |
| Genadvance® Life: Clariant, INCI = Polyquaternium-116 und Butylene Glycol<br>Genadvance® Repair: Clariant, INCI = Quaternium-98<br>Genadvance® Hydra: Clariant, INCI = Lauryl/Myristyl Polyricinoleate & Glycerin | | | | | | |

| Example | 28m | 28n | 28o | 28p | 28q | 28r |
|---|---|---|---|---|---|---|
| **Ingredient** | **wt.-%** | **wt.-%** | **wt.-%** | **wt.-%** | **wt.-%** | **wt.-%** |
| Multilamellar vesicles comprising fragrance | 1.0 | 1.0 | 1.0 | 1.0 | 1.0 | 1.0 |
| Cetearyl Alcohol | 4.0 | 4.0 | 4.0 | 4.0 | 4.0 | 4.0 |
| CTAC | - | - | - | 0.2 | 0.2 | 0.2 |
| BTAC | - | - | - | - | - | - |
| BTMS | 0.5 | 0.5 | 0.5 | - | - | - |
| Genadvance® Life | 1.5 | - | - | 1.8 | - | - |
| Genadvance® Repair | - | 1.5 | - | - | 1.8 | - |
| Genadvance® Hydra | - | - | 1.5 | - | - | 1.8 |
| Xiameter PMX-200 (dimethicone) | 0.2 | 0.2 | 0.2 | 0.2 | 0.2 | 0.2 |
| Preservative | q.s. | q.s. | q.s. | q.s. | q.s. | q.s. |
| Water | Ad. 100 | Ad. 100 | Ad. 100 | Ad. 100 | Ad. 100 | Ad. 100 |
| Genadvance® Life: Clariant, INCI = Polyquaternium-116 und Butylene Glycol<br>Genadvance® Repair: Clariant, INCI = Quaternium-98<br>Genadvance® Hydra: Clariant, INCI = Lauryl/Myristyl Polyricinoleate & Glycerin | | | | | | |

| Example | 28s | 28t | 28u | 28v | 28w | 28x |
|---|---|---|---|---|---|---|
| **Ingredient** | **wt.-%** | **wt.-%** | **wt.-%** | **wt.-%** | **wt.-%** | **wt.-%** |
| Multilamellar vesicles comprising fragrance | 1.0 | 1.0 | 1.0 | 1.0 | 1.0 | 1.0 |
| Cetearyl Alcohol | 4.0 | 4.0 | 4.0 | 4.0 | 4.0 | 4.0 |
| CTAC | - | - | - | - | - | - |
| BTAC | 0.2 | 0.2 | 0.2 | - | - | - |
| BTMS | - | - | - | 0.2 | 0.2 | 0.2 |
| Genadvance® Life | 1.8 | - | - | 1.8 | - | - |

(continued)

| Example | 28s | 28t | 28u | 28v | 28w | 28x |
|---|---|---|---|---|---|---|
| **Ingredient** | **wt.-%** | **wt.-%** | **wt.-%** | **wt.-%** | **wt.-%** | **wt.-%** |
| Genadvance® Repair | - | 1.8 | - | - | 1.8 | - |
| Genadvance® Hydra | - | - | 1.8 | - | - | 1.8 |
| Xiameter PMX-200 (dimethicone) | 0.2 | 0.2 | 0.2 | 0.2 | 0.2 | 0.2 |
| Preservative | q.s. | q.s. | q.s. | q.s. | q.s. | q.s. |
| Water | Ad. 100 | Ad. 100 | Ad. 100 | Ad. 100 | Ad. 100 | Ad. 100 |
| Genadvance® Life: Clariant, INCI = Polyquaternium-116 und Butylene Glycol<br>Genadvance® Repair: Clariant, INCI = Quaternium-98<br>Genadvance® Hydra: Clariant, INCI = Lauryl/Myristyl Polyricinoleate & Glycerin | | | | | | |

Example 29: A shampoo composition

| Ingredient | Active level, wt% |
|---|---|
| Aqua | To 100 |
| Multilamellar vesicles comprising fragrance | 1 |
| sodium laureth sulfate | 8 |
| sodium lauryl sulfate | 8 |
| cocamide MIPA | 4 |
| glycerin | 3.5 |
| glycol distearate | 3 |
| sodium chloride | 2.5 |
| dimethicone | 2 |
| Pyrus malus extract | 1.5 |
| PPG-5-ceteth-20 | 1.5 |
| salicylic acid | 1 |
| Hair conditioning agent | 1 |
| carbomer | 0.4 |
| niacinamide | 0.2 |
| pyridoxine HCl | 0.2 |
| guar hydroxypropyltrimonium chloride | 0.2 |
| Saccharum officinarum extract | 0.2 |
| hydrolyzed soy protein | 0.2 |
| methyl cocoate | 0.15 |
| sodium cocoate | 0.15 |
| Preservative | As needed |
| citric acid | As needed, pH 4.5 - 6.5 |
| sodium hydroxide | As needed, pH 4.5 - 6.5 |
| Colorant | As needed |

Example 30: A shampoo composition

| Ingredient | Active level, wt% |
|---|---|
| Aqua | To 100 |
| Multilamellar vesicles comprising fragrance | 1 |
| sodium laureth sulfate | 15 |
| dimethicone | 3 |
| glycol distearate | 3 |
| coco-betaine | 2 |
| guar hydroxypropyltrimonium chloride | 1.5 |
| Hair conditioning agent | 1.5 |
| niacinamide | 0.5 |
| cocamide MIPA | 0.5 |
| saccharum officinarum extract | 0.5 |
| sodium chloride | 0.5 |
| hydroxypropyltrimonium hydrolyzed wheat protein | 0.4 |
| sodium cocoate | 0.3 |
| aminopropyl triethoxysilane | 0.25 |
| salicylic acid | 0.25 |
| caprylic/capric triglyceride | 0.2 |
| carbomer | 0.2 |
| disodium cocoamphodiacetate | 0.15 |
| potassium chloride | 0.15 |
| methyl cocoate | 0.1 |
| Preservative | As needed |
| citric acid | As needed, pH 4.5 - 6.5 |
| sodium hydroxide | As needed, pH 4.5 - 6.5 |
| Colorant | As needed |

Example 31: A shampoo composition

| Ingredient | Active level, wt% |
|---|---|
| Aqua | To 100 |
| Multilamellar vesicles comprising fragrance | 1 |
| sodium laureth sulfate | 14 |
| coco-betaine | 4 |
| glycerin | 3 |
| glycol distearate | 2.5 |

(continued)

| Ingredient | Active level, wt% |
|---|---|
| dimethicone | 2 |
| Hair conditioning agent | 1.5 |
| niacinamide | 0.5 |
| coconut oil | 0.5 |
| saccharum officinarum extract | 0.5 |
| sodium chloride | 0.5 |
| olive fruit oil | 0.4 |
| PPG-5-ceteth-20 | 0.3 |
| trideceth-6 | 0.25 |
| polyquaternium-6 | 0.25 |
| salicylic acid | 0.25 |
| amodimethicone | 0.2 |
| carbomer | 0.2 |
| cetrimonium chloride | 0.15 |
| Preservative | As needed |
| citric acid | As needed, pH 4.5 - 6.5 |
| sodium hydroxide | As needed, pH 4.5 - 6.5 |
| Colorant | As needed |

Example 32: A shampoo composition

| Ingredient | Active level, wt% |
|---|---|
| Aqua | To 100 |
| Multilamellar vesicles comprising fragrance | 1 |
| ammonium lauryl sulfate | 16 |
| cocoamidopropyl betaine | 5 |
| sodium chloride | 2.5 |
| niacinamide | 2 |
| Saccharum officinarum extract | 1 |
| Hair conditioning agent | 1 |
| hydroxypropyl guar hydroxypropyltrimonium chloride | 0.8 |
| salicylic acid | 0.3 |
| hexylene glycol | 0.1 |
| Preservative | As needed |
| citric acid | As needed, pH 4.5 - 6.5 |
| sodium hydroxide | As needed, pH 4.5 - 6.5 |
| Colorant | As needed |

Example 33: A shampoo composition

| Ingredient | Active level, wt% |
|---|---|
| Aqua | To 100 |
| Multilamellar vesicles comprising fragrance | 1 |
| sodium laureth sulfate | 16 |
| glycol distearate | 4 |
| coco-betaine | 2.5 |
| glycerin | 2 |
| sodium chloride | 1.5 |
| dimethicone | 1.5 |
| Hair conditioning agent | 1.5 |
| niacinamide | 1 |
| guar hydroxypropyltrimonium chloride | 0.8 |
| cocamide MIPA | 0.5 |
| Saccharum officinarum extract | 0.3 |
| PPG-5-ceteth-20 | 0.2 |
| salicylic acid | 0.2 |
| fumaric acid | 0.15 |
| carbomer | 0.15 |
| pyridoxine HCl | 0.1 |
| Preservative | As needed |
| citric acid | As needed, pH 4.5 - 6.5 |
| sodium hydroxide | As needed, pH 4.5 - 6.5 |
| Colorant | As needed |

Example 34: A shampoo composition

| Ingredient | Active level, wt% |
|---|---|
| Aqua | To 100 |
| Multilamellar vesicles comprising fragrance | 1 |
| sodium laureth sulfate | 16 |
| coco-betaine | 4 |
| niacinamide | 2 |
| Ribes nigrum oil | 1.5 |
| cocamide MIPA | 1.5 |
| sodium chloride | 1.5 |
| Hair conditioning agent | 1.2 |

(continued)

| Ingredient | Active level, wt% |
|---|---|
| sodium cocoate | 0.8 |
| cocamide MIPA | 0.5 |
| Olea europaea oil (olive) fruit oil | 0.3 |
| PEG-55 propylene glycol oleate | 0.3 |
| PEG-60 hydrogenated castor oil | 0.3 |
| polyquaternium-7 | 0.2 |
| salicylic acid | 0.15 |
| amodimethicone | 0.15 |
| propylene glycol | 0.1 |
| Butyrospermum parkii butter | 0.1 |
| laureth-5 carboxylic acid | 0.1 |
| Preservative | As needed |
| citric acid | As needed, pH 4.5 - 6.5 |
| sodium hydroxide | As needed, pH 4.5 - 6.5 |
| Colorant | As needed |

Example 35: A shampoo composition

| Ingredient | Active level, wt% |
|---|---|
| Aqua | To 100 |
| Multilamellar vesicles comprising fragrance | 1 |
| sodium laureth sulfate | 10 |
| disodium cocamphodiacetate | 5 |
| glycerin | 3 |
| glycol distearate | 2 |
| sodium chloride | 1.5 |
| Hair conditioning agent | 1.5 |
| cocamide MEA | 1 |
| polyquaternium-10 | 1 |
| salicylic acid | 0.5 |
| carbomer | 0.3 |
| Preservative | As needed |
| citric acid | As needed, pH 4.5 - 6.5 |
| sodium hydroxide | As needed, pH 4.5 - 6.5 |
| Colorant | As needed |

Example 36: A shampoo composition

| Ingredient | Active level, wt% |
|---|---|
| Aqua | To 100 |
| Multilamellar vesicles comprising fragrance | 1 |
| sodium laureth sulfate | 12 |
| coco-betaine | 3 |
| cocamide MIPA | 2 |
| sodium chloride | 1.8 |
| Olea europaea (olive) fruit oil | 0.5 |
| Hair conditioning agent | 0.5 |
| PPG-5-ceteth-20 | 0.4 |
| PEG-55 propylene glycol oleate | 0.4 |
| PEG-60 hydrogenated castor oil | 0.25 |
| polyquaternium-10 | 0.25 |
| salicylic acid | 0.2 |
| amodimethicone | 0.15 |
| propylene glycol | 0.15 |
| laureth-5 | 0.1 |
| Preservative | As needed |
| citric acid | As needed, pH 4.5 - 6.5 |
| sodium hydroxide | As needed, pH 4.5 - 6.5 |
| Colorant | As needed |

Example 37: A shampoo composition

| Ingredient | Active level, wt% |
|---|---|
| Aqua | To 100 |
| Multilamellar vesicles comprising fragrance | 1 |
| sodium laureth sulfate | 14 |
| coco-betaine | 3 |
| laureth-5 carboxylic acid | 2.5 |
| cocamide MIPA | 2 |
| amodimethicone | 1.5 |
| sodium chloride | 1.5 |
| Hair conditioning agent | 1 |
| polyquaternium-10 | 0.5 |
| PPG-5-ceteth-20 | 0.5 |

(continued)

| Ingredient | Active level, wt% |
|---|---|
| PEG-55 propylene glycol oleate | 0.3 |
| PEG-60 hydrogenated castor oil | 0.3 |
| coconut oil | 0.2 |
| Preservative | As needed |
| citric acid | As needed, pH 4.5 - 6.5 |
| sodium hydroxide | As needed, pH 4.5 - 6.5 |
| Colorant | As needed |

Example 38: A shampoo composition

| Ingredient | Active level, wt% |
|---|---|
| Aqua | To 100 |
| Multilamellar vesicles comprising fragrance | 1 |
| sodium laureth sulfate | 15 |
| coco-betaine | 3 |
| glycerin | 3 |
| glycol distearate | 2.5 |
| sodium chloride | 2 |
| amodimethicone | 2 |
| PPG-5-ceteth-20 | 1.5 |
| dimethicone | 1 |
| Hair conditioning agent | 0.8 |
| polyquaternium-6 | 0.4 |
| carbomer | 0.4 |
| arginine | 0.1 |
| glutamic acid | 0.1 |
| trideceth-6 | 0.1 |
| hydroxypropyltrimonium hydrolyzed wheat protein | 0.1 |
| cetrimonium chloride | 0.1 |
| Preservative | As needed |
| citric acid | As needed, pH 4.5 - 6.5 |
| sodium hydroxide | As needed, pH 4.5 - 6.5 |
| Colorant | As needed |

Example 39: A shampoo composition

| Ingredient | Active level, wt% |
|---|---|
| Aqua | To 100 |
| Multilamellar vesicles comprising fragrance | 1 |
| ammonium lauryl sulfate | 15 |
| cocamidopropyl betaine | 3 |
| sodium chloride | 2 |
| hexylene glycol | 1.8 |
| hydroxypropyl guar hydroxypropyltrimonium chloride | 1.5 |
| Hair conditioning agent | 1 |
| Preservative | As needed |
| citric acid | As needed, pH 4.5 - 6.5 |
| sodium hydroxide | As needed, pH 4.5 - 6.5 |
| Colorant | As needed |

Example 40: A shampoo composition

| Ingredient | Active level, wt% |
|---|---|
| Aqua | To 100 |
| Multilamellar vesicles comprising fragrance | 1 |
| sodium cocoyl isethionate | 4 |
| sodium lauryl sulfoacetate | 3 |
| disodium laureth sulfosuccinate | 2.5 |
| cocamidopropyl betaine | 2.5 |
| sodium lauroyl sarcosinate | 2.5 |
| glycol distearate | 2 |
| glycereth-26 | 1.8 |
| decyl glucoside | 1.5 |
| hydrogenated coconut acid | 1.4 |
| PPG-5-ceteth-20 | 1.3 |
| divinyldimethicone/dimethicone copolymer | 1.2 |
| sodium chloride | 1.1 |
| amodimethicone | 1 |
| Hair conditioning agent | 1 |
| polyquaternium-7 | 1 |
| polyquaternium-10 | 0.7 |
| sodium isethionate | 0.5 |
| PEG-55 propylene glycol oleate | 0.4 |
| propylene glycol | 0.4 |

(continued)

| Ingredient | Active level, wt% |
|---|---|
| carbomer | 0.4 |
| C11-15 pareth-7 | 0.3 |
| glycerin | 0.3 |
| trideceth-12 | 0.25 |
| laureth-9 | 0.2 |
| hydroxypropyltrimonium hydrolyzed wheat protein | 0.1 |
| Preservative | As needed |
| citric acid | As needed, pH 4.5 - 6.5 |
| sodium hydroxide | As needed, pH 4.5 - 6.5 |
| Colorant | As needed |

Example 41: A shampoo composition

| Ingredient | Active level, wt% |
|---|---|
| Aqua | To 100 |
| Multilamellar vesicles comprising fragrance | 1 |
| sodium laureth sulfate | 10 |
| disodium cocoamphodiacetate | 4 |
| disodium laureth sulfosuccinate | 2.5 |
| sodium chloride | 2 |
| glycol distearate | 1.8 |
| zinc pyrithione | 1.5 |
| PPG-5-ceteth-20 | 1 |
| carbomer | 0.6 |
| Hair conditioning agent | 0.5 |
| polyquaternium-10 | 0.4 |
| Preservative | As needed |
| citric acid | As needed, pH 4.5 - 6.5 |
| sodium hydroxide | As needed, pH 4.5 - 6.5 |
| Colorant | As needed |

Example 42: A shampoo composition

| Ingredient | Active level, wt% |
|---|---|
| Aqua | To 100 |
| Multilamellar vesicles comprising fragrance | 1 |

(continued)

| Ingredient | Active level, wt% |
|---|---|
| sodium methyl cocoyl taurate | 5 |
| laureth-5 carboxylic acid | 4 |
| sodium chloride | 2 |
| cocamidopropyl betaine | 2 |
| glycerin | 1.8 |
| glycol distearate | 1.5 |
| Hair conditioning agent | 1.5 |
| polyquaternium-10 | 1 |
| PPG-5 ceteth-20 | 0.5 |
| sodium lauroyl glutamate | 0.4 |
| propylene glycol | 0.2 |
| PEG-55 propylene glycol oleate | 0.15 |
| Argania spinosa kernel oil | 0.1 |
| Preservative | As needed |
| citric acid, lactic acid | As needed, pH 4.5 - 6.5 |
| sodium hydroxide | As needed, pH 4.5 - 6.5 |
| Colorant | As needed |

Example 43: A shampoo composition

| Ingredient | Active level, wt% |
|---|---|
| Aqua | To 100 |
| Multilamellar vesicles comprising fragrance | 1 |
| sodium laureth sulfate | 10 |
| disodium cocoamphodiacetate | 4 |
| glycol distearate | 2 |
| sodium chloride | 1.5 |
| Hair conditioning agent | 1.3 |
| carbomer | 0.4 |
| diaminopyrimidine oxide | 0.3 |
| disodium ricinoleamido MEA sulfosuccinate | 0.3 |
| hexylene glycol | 0.2 |
| panthenol | 0.1 |
| polyquaternium-10 | 0.1 |
| Preservative | As needed |
| citric acid | As needed, pH 4.5 - 6.5 |
| sodium hydroxide, ammonium hydroxide | As needed, pH 4.5 - 6.5 |

(continued)

| Ingredient | Active level, wt% |
|---|---|
| Colorant | As needed |

Example 44: A shampoo composition

| Ingredient | Active level, wt% |
|---|---|
| Aqua | To 100 |
| Multilamellar vesicles comprising fragrance | 1 |
| ammonium lauryl sulfate | 12 |
| cocamidopropyl betaine | 3 |
| sodium chloride | 1.5 |
| Hair conditioning agent | 1.3 |
| sodium chloride | 1 |
| alcohol denat. | 0.8 |
| diethyllutidinate | 0.3 |
| hydroxypropyl guar hydroxypropyltrimonium chloride | 0.2 |
| polyquaternium-30 | 0.15 |
| Preservative | As needed |
| citric acid | As needed, pH 4.5 - 6.5 |
| sodium hydroxide | As needed, pH 4.5 - 6.5 |
| Colorant | As needed |

Example 45: A shampoo composition

| Ingredient | Active level, wt% |
|---|---|
| Aqua | To 100 |
| Multilamellar vesicles comprising fragrance | 1 |
| sodium laureth sulfate | 12 |
| PEG-200 hydrogenated glyceryl palmate | 4 |
| disodium cocoamphodiacetate | 2.5 |
| polysorbate 20 | 2 |
| glycerin | 1.5 |
| PEG-7 glyceryl cocoate | 1.4 |
| disodium ricinoleamido MEA-sulfosuccinate | 1 |
| Hair conditioning agent | 1 |
| polyquaternium-10 | 0.6 |
| Preservative | As needed |

(continued)

| Ingredient | Active level, wt% |
|---|---|
| citric acid | As needed, pH 4.5 - 6.5 |
| sodium hydroxide | As needed, pH 4.5 - 6.5 |
| Colorant | As needed |

Example 46: A shampoo composition

| Ingredient | Active level, wt% |
|---|---|
| Aqua | To 100 |
| Multilamellar vesicles comprising fragrance | 1 |
| sodium cocoyl isethionate | 4.5 |
| sodium lauryl sulfoacetate | 3 |
| disodium laureth sulfosuccinate | 2.5 |
| cocamidopropyl betaine | 2.5 |
| sodium lauroyl sarcosinate | 2.3 |
| glycol distearate | 2 |
| glycereth-26 | 1.8 |
| decyl glucoside | 1.7 |
| hydrogenated coconut acid | 1.5 |
| PPG-5-ceteth-20 | 1.5 |
| PEG-55 propylene glycol oleate | 1.5 |
| propylene glycol | 1.5 |
| sodium chloride | 1.5 |
| divinyldimethicone/dimethicone copolymer | 1 |
| polyquaternium-7 | 1 |
| amodimethicone | 1 |
| Hair conditioning agent | 1 |
| polyquaternium-10 | 0.8 |
| sodium isethionate | 0.6 |
| carbomer | 0.6 |
| C11-15 pareth-7 | 0.4 |
| glycerin | 0.3 |
| laureth-9 | 0.2 |
| Hibiscus esculentus | 0.1 |
| Preservative | As needed |
| citric acid | As needed, pH 4.5 - 6.5 |
| sodium hydroxide | As needed, pH 4.5 - 6.5 |
| Colorant | As needed |

Example 47: A shampoo composition

| Ingredient | Active level, wt% |
|---|---|
| Aqua | To 100 |
| Multilamellar vesicles comprising fragrance | 1 |
| cocamidopropyl betaine | 5 |
| sodium lauryl sulfoacetate | 4 |
| disodium laureth sulfosuccinate | 3.5 |
| sodium lauroyl sarcosinate | 3 |
| glycol distearate | 2 |
| sodium chloride | 1.7 |
| decyl glucoside | 1.5 |
| Hair conditioning agent | 1.5 |
| polyquaternium-10 | 1 |
| PPG-5-ceteth-20 | 1 |
| coco-betaine | 0.8 |
| PEG-55 propylene glycol oleate | 0.8 |
| propylene glycol | 0.5 |
| salicylic acid | 0.4 |
| carbomer | 0.4 |
| Preservative | As needed |
| citric acid | As needed, pH 4.5 - 6.5 |
| sodium hydroxide | As needed, pH 4.5 - 6.5 |
| Colorant | As needed |

Example 48: A shampoo composition

| Ingredient | Active level, wt% |
|---|---|
| Aqua | To 100 |
| Multilamellar vesicles comprising fragrance | 1 |
| sodium laureth sulfate | 10 |
| PEG-200 hydrogenated glyceryl palmate | 3 |
| disodium cocoamphodiacetate | 2.5 |
| polysorbate-20 | 2 |
| sodium chloride | 1.7 |
| Hair conditioning agent | 1 |
| glycerin | 1 |
| PEG-7 glyceryl cocoate | 1 |

(continued)

| Ingredient | Active level, wt% |
|---|---|
| polysorbate 21 | 0.5 |
| polyquaternium-10 | 0.2 |
| disodium EDTA | 0.2 |
| Preservative | As needed |
| citric acid | As needed, pH 4.5 - 6.5 |
| sodium hydroxide | As needed, pH 4.5 - 6.5 |
| Colorant | As needed |

Example 49: A shampoo composition

| Ingredient | Active level, wt% |
|---|---|
| Aqua | To 100 |
| Multilamellar vesicles comprising fragrance | 1 |
| sodium laureth sulfate | 15 |
| coco-betaine | 3 |
| glycol distearate | 2.5 |
| sodium chloride | 2 |
| PPG-5-ceteth-20 | 1.8 |
| Hair conditioning agent | 1.5 |
| piroctone olamine | 1.5 |
| octyldodecanol | 1 |
| carbomer | 1 |
| guar hydroxypropyltrimonium chloride | 1 |
| sodium hyaluronate | 0.4 |
| Preservative | As needed |
| citric acid | As needed, pH 4.5 - 6.5 |
| sodium hydroxide | As needed, pH 4.5 - 6.5 |
| Colorant | As needed |

Example 50: A shampoo composition

| Ingredient | Active level, wt% |
|---|---|
| Aqua | To 100 |
| Multilamellar vesicles comprising fragrance | 1 |
| sodium laureth sulfate | 16 |
| dimethicone | 3 |

(continued)

| Ingredient | Active level, wt% |
|---|---|
| coco-betaine | 1.5 |
| glycol distearate | 1.5 |
| sodium chloride | 1.5 |
| guar hydroxypropyltrimonium chloride | 1 |
| cocamide MIPA | 1 |
| Hair conditioning agent | 1 |
| sodium cocoate | 0.8 |
| benzophenone-4 | 0.7 |
| carbomer | 0.6 |
| Preservative | As needed |
| citric acid | As needed, pH 4.5 - 6.5 |
| sodium hydroxide | As needed, pH 4.5 - 6.5 |
| Colorant | As needed |

Example 51: A shampoo composition

| Ingredient | Active level, wt% |
|---|---|
| Aqua | To 100 |
| Multilamellar vesicles comprising fragrance | 1 |
| sodium laureth sulfate | 12 |
| coco-glucoside | 4 |
| sodium chloride | 2 |
| distearyl ether | 1.5 |
| behenyl alcohol | 1.5 |
| PPG-5-ceteth-20 | 1 |
| aminomethyl propanol | 1 |
| Hair conditioning agent | 1 |
| polysilicone-8 | 0.8 |
| polyquaternium-16 | 0.8 |
| carbomer | 0.5 |
| Preservative | As needed |
| citric acid | As needed, pH 4.5 - 6.5 |
| sodium hydroxide | As needed, pH 4.5 - 6.5 |
| Colorant | As needed |

## Claims

1. A composition, in particular cosmetic composition or fabric conditioner composition, comprising multilamellar vesicles

and one or more further components (F),
wherein the multilamellar vesicles are in the shape of a rotational body and comprise one or more (preferably two or more) concentric lipid double layers and fragrance,
wherein the multilamellar vesicles have a mean diameter between 100 and 800 nm, and
wherein the lipid double layers comprise

a) at least one surfactant having a HLB value of greater than 6, and
b) an amphiphilic compound having a logP value of 1 or above, and

wherein the multilamellar vesicles comprise in addition to components a) and b) a fragrance having a logP value of 1 or above.

**2.** The composition according to claim 1, wherein the multilamellar vesicles further comprise at least one co-surfactant as component c), which is preferably selected from the group consisting of sorbitan esters, citric esters, lactic esters, partial fatty acid glycerides, polyglycerides, glycerol esters, polyglycerol esters, sorbitol esters, fatty alcohols, propylene glycol esters, methyl glucoside ester, alkyl polyglucosides, sugar esters or combinations of two or more thereof.

**3.** The composition according to claim 1 or 2, wherein the multilamellar vesicles further comprise at least one wax as component d), which is preferably a wax of the monoester type.

**4.** The composition according to any of claims 1 to 3, wherein component a) comprises a nonionic, cationic, anionic and/or amphoteric surfactant.

**5.** The composition according to any of claims 1 to 4, wherein component a) comprises a nonionic surfactant selected from the group consisting of polyoxyethylene sorbitan esters, polyoxyethylene sorbitol esters, polyoxyalkylene fatty alcohol ethers, polyoxyalkylene fatty acid esters, alkoxylated glycerides, polyoxyethylene methyl glucoside esters, alkyl polyglucosides, EO-PO blockpolymers or combinations of two or more thereof, or wherein the anionic surfactant is selected from the group consisting of sulfonates of alkylbenzenesulfonates, alkanesulfonates, olefinsulfonates, alkyl ether sulfates, alkyl sulfates, sulfosuccinates, alkyl phosphates, alkyl ether phosphates, protein fatty acid condensates, amino acid-based surfactants, isethionates, taurides, acyl lactylates, neutralized fatty acids or combinations of two or more thereof, or wherein the cationic surfactants are selected from the group consisting of esterquats, ditallow dimethyl ammonium chloride, C12/14 alkyl dimethyl benzyl ammonium chloride, alkyl dimethyl benzil ammonium chloride, cetyl trimethyl ammonium chloride, stearyl trimethyl ammonium chloride, behenyl trimethyl ammonium chloride, alkyl hydroxyethyl dimethyl ammonium chloride, distearyl dimethyl ammonium chloride, dihydrogenated tallow fatty alkyl dimethyl ammonium chloride or combinations of two or more thereof.

**6.** The composition according to any of claims 1 to 5, wherein component b) comprises an ester of a fatty acid, preferably a triglyceride of fatty acids and/or an ester of a fatty acid and a fatty alcohol, most preferred a triglyceride of one or more fatty acids having 8 to 24 carbon atoms and/or an ester of a fatty acid having 8 to 24 carbon atoms with a fatty alcohol having 8 to 24 carbon atoms.

**7.** The composition according to any of claims 1 to 6, wherein the multilamellar vesicles comprise as component e) at least one amphiphilic copolymer, preferably polyvinyl acetate, polyvinyl pyrrolidone, polyvinyl alcohols, vinyl pyrrolidone/hexadecene copolymer, vinyl pyrrolidone/eicosene copolymer, silicone oils or their derivatives.

**8.** The composition according to any of claims 1 to 7, wherein the composition comprises from 0.1 to 20 wt.-%, preferably from 0.2 to 15 wt.-%, more preferably from 0.3 to 10 wt.-%, even more preferably from 0.5 to 5 wt.-%, particularly preferably from 1 to 3 wt.-% of the multilamellar vesicles, based on the total weight of the composition.

**9.** The composition according to any of claims 1 to 8, wherein the composition is a cosmetic composition, preferably a hair care composition or a skin care composition, particularly preferably a shampoo composition or a hair conditioner composition.

**10.** The composition according to any of claims 1 to 8, wherein the composition is a fabric conditioner composition.

**11.** The composition according to any of claims 1 to 10, wherein the composition comprises as further component (F) a cationic compound, preferably a cationic ammonium compound.

**12.** The composition according to any of claims 1 to 11, wherein the composition comprises as further component (F) a rheology modifying agent, preferably a gelling and/or thickening agent, more preferably a thickening polymer.

**13.** The composition according to any of claims 1 to 12, wherein the composition comprises as further component (F) a nonionic, cationic, anionic and/or amphoteric surfactant.

**14.** The composition according to any of claims 1 to 9 and 11 to 13, wherein the composition is a shampoo composition comprising

(i) from 0.3 wt.-% to 10 wt.-% of the multilamellar vesicles;
(ii) from 5 wt.-% to 20 wt.-% of one or more anionic surfactants;
(iii) at least 50 wt.-% water; and
(iv) at least one further cosmetically acceptable component (F) selected from the group consisting of silicone, cationic polymer, rheology modifiying agent, and an amphoteric or zwitterionic surfactant.

**15.** The composition according to any of claims 1 to 8 and 10 to 13, wherein the composition is a fabric conditioner composition comprising

(i) from 0.3 wt.-% to 10 wt.-% of the multilamellar vesicles;
(ii) from 10 wt.-% to 50 wt.-%, preferably from 20 wt.-% to 35 wt.-% of one or more cationic softening agents, preferably quaternary ammonium compounds;
(iii) at least 50 wt.-% water; and
(iv) at least one further component (F) selected from the group consisting of hydrophobic agents, non-ionic alkoxylated materials, polymeric thickening agents, non-ionic softeners, co-softeners, fatty complexing agents.

Figure 1

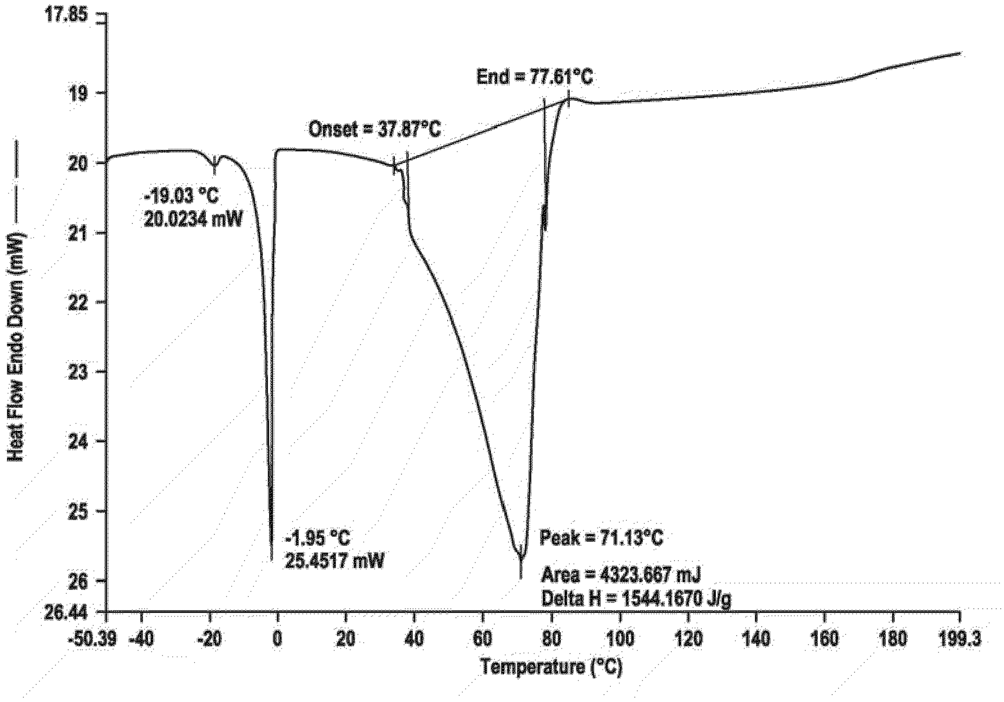
17.85
19
20
21
22
23
24
25
26
26.44
Heat Flow Endo Down (mW)
-50.39 -40 -20 0 20 40 60 80 100 120 140 160 180 199.3
Temperature (°C)
-19.03 °C
20.0234 mW
-1.95 °C
25.4517 mW
Onset = 37.87°C
End = 77.61°C
Peak = 71.13°C
Area = 4323.667 mJ
Delta H = 1544.1670 J/g

Figure 2

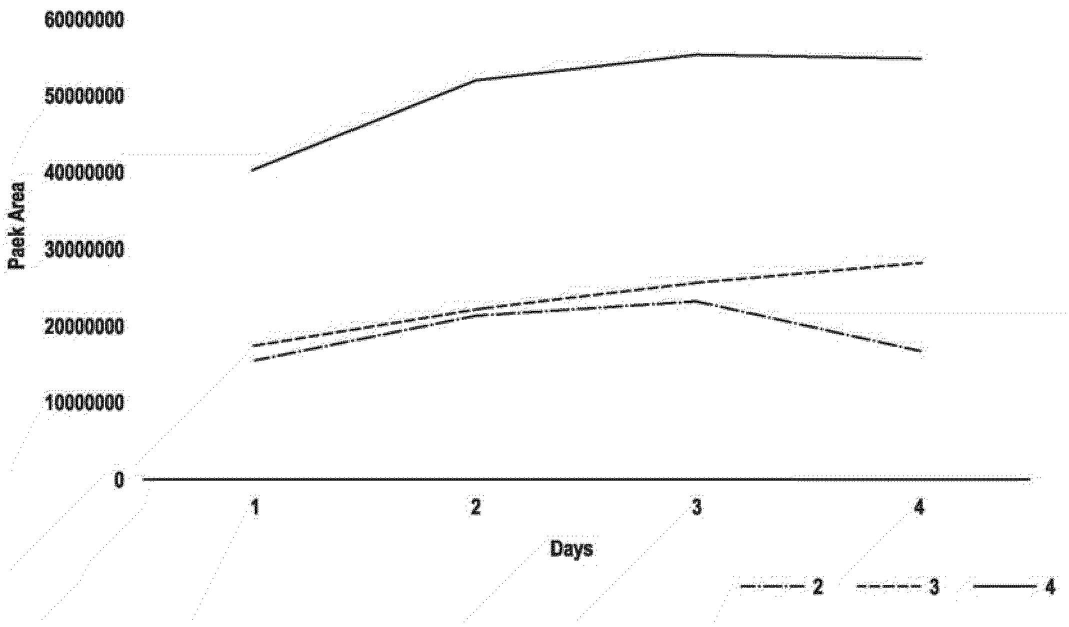
60000000
50000000
40000000
30000000
20000000
10000000
0
Paek Area
1
2
3
4
Days
2
3
4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019115621 A1
- US 20130201785 A1
- US 4529773 A, Witiak
- US 2809971 A
- US 3236733 A
- US 3753196 A
- US 3761418 A
- US 4345080 A
- US 4323683 A
- US 4379753 A
- US 4470982 A
- US 4009256 A1
- WO 9522311 A1
- US 3962418 A
- US 3958581 A
- WO 9206154 A
- US 5194639 A
- WO 2013178700 A
- EP 0550637 A
- WO 9631188 A
- EP 0530974 A
- US 4137180 A
- WO 2010078959 A
- EP 343840 A
- US 20020132749 A
- US 429116 A
- US 4386213 A
- AU 1441688
- WO 0146361 A
- WO 2012072368 A


### Non-patent literature cited in the description


- *Journal of the Society of Cosmetic Chemists,* 1954, vol. 5 (4), 249-256
- Gas/Liquid and Liquid/Liquid Interfaces. Proceedings of 2nd International Congress Surface Activity. Butterworths, 1957, 426-438
- **A. LEO ; C. HANSCH ; D. ELKINS.** *Chemical Reviews,* 1971, vol. 71 (6
- International Cosmetic Ingredient Dictionary and Handbook. 2014
- CTFA Cosmetic Ingredient Handbook. 1992
- International Cosmetic Ingredient Dictionary and Handbook
- **JOHNSON ; SHOOLERY.** *Anal. Chem.,* 1962, vol. 34, 1136